# EUROPEAN PATENT SPECIFICATION

(11) **EP 2 596 743 B1**
(45) Date of publication and mention of the grant of the patent: **31.12.2014**
(21) Application number: 13156521.0
(22) Date of filing: 18.11.2009
(51) Int. Cl.: A61B 1/005, A61B 1/018, A61B 1/233

(54) **Endoscope system and assist device**
Endoskopsystem und Unterstützungsvorrichtung dafür
Système endoscope et dispositif d'assistance

(30) Priority: 19.11.2008 JP 2008295577; 03.03.2009 JP 2009049372
(43) Date of publication of application: 29.05.2013
(62) Divisional of application: 09252646.6
(73) Proprietor: Fujifilm Corporation, Minato-ku Tokyo (JP)
(72) Inventor: Torii, Yuichi, Saitama-shi, Saitama (JP); Arai, Haruhiko, Saitama-shi, Saitama (JP); Koga, Takehiko, Saitama-shi, Saitama (JP); Ikeda, Toshiyuki, Saitama-shi, Saitama (JP); Inoue, Masaya, Saitama-shi, Saitama (JP); Seki, Masahiro, Saitama-shi, Saitama (JP); Nishino, Tomoharu, Saitama-shi, Saitama (JP); Iyama, Shozo, Saitama-shi, Saitama (JP)
(74) Representative: Stevens, Jason Paul

(56) References cited:
- US-A1- 2006 111 612
- US-B1- 6 878 106

## Description

### BACKGROUND OF THE INVENTION

### 1. Field of the Invention

The present invention relates to an endoscope system and an assist device. More particularly, the present invention relates to an endoscope system in which a nasal type of an endoscope is used for medical treatment, and an assist device for use in combination with the nasal endoscope.

### 2. Description Related to the Prior Art

A nasal endoscope is known in the field of medical instruments, and has an elongated tube with a smaller diameter than that of an oral endoscope. Examples of the nasal endoscope are disclosed in JP-A 2006-068030 and 2007-061377. There are several advantages of examination with the nasal endoscope over the oral endoscope. The nasal endoscope does not contact a tongue root of a patient or a surface of his or her throat, and can reduce physical stress of the patient because his or her nausea and strange feeling can be reduced. Anaesthetic drug for use can be a smaller amount than in the oral endoscope. Also, the patient can talk with a doctor or other staff even during the examination. The patient is enabled to breathe orally.

In the same manner as the oral endoscope, the nasal endoscope includes imaging optics and lighting optics incorporated in a distal portion or head assembly of the elongated tube for entry in the patient's body. An object in the body is illuminated by the lighting optics. An image of an object is picked up through the imaging optics, to produce an image signal. A display panel is driven to display the image. Various elements are contained in the elongated tube its tube lumen extending from the distal portion to a proximal portion, including an instrument channel (operable also as a suction channel), air/water supply channel, light guide optics and the like.

The light guide optics include an entrance end and an exit end for entry and exit of light, and guide light from the light source apparatus to the distal portion. The light from the exit end is passed through a lens, and is applied to an object through a lighting window formed in a distal end surface in the distal portion. A distal opening is a first end of the instrument channel and open in the distal end surface. A proximal opening is a second of the instrument channel and open is a proximal portion. A treatment instrument such as forceps device or other medical instrument is inserted in the instrument channel through the proximal opening, and guided toward the distal opening.

The endoscope of a nasal type is characterized in having higher flexibility of the elongated tube than the oral endoscope for the purpose of passage in a tortuous path from an external nostril to a middle or lower nasal tract. A diameter of the elongated tube is as small as 5 - 6 mm in contrast with that of the elongated tube of the oral endoscope (approximately 9 mm). The kinds and number of the treatment instrument or medical instruments that can enter the instrument channel may be restricted, because the diameter of the bore of the instrument channel in the elongated tube, which is as small as approximately 2 mm. There are problems in difficulties in the treatment, for example, cutting of polyp (polypectomy and mucosectomy) in a stomach or oesophagus, tissue clamping (haemostasis) of bleeding tissue of a body part by use of medical clips, with a gastric ulcer of the like.

To solve such problems, a combined of use of an assist device is conceivable in combination with the endoscope. The assist device has the elongated tube of which a diameter is equal to or smaller than that of the endoscope. The assist device has the instrument channel contained in the elongated tube and having a greater bore than that of the instrument channel. For example, the elongated tube of the assist device is entered through a second nostril, and used in combination with the endoscope. This is for the purpose of providing an additional function to the endoscope.

A problem arises in the combined use of the endoscope with the assist device. Operability of the assist device is not good, as the distal portions of the elongated tubes which should be manipulated are discretely entered in the body. For the medical treatment by their combined use, it is conceivable to retain the distal portions of the elongated tubes on one another within a body cavity of the patient after discrete initial entry of the elongated tubes through the external nostrils, particularly in an area of the body cavity from the lower nasal tract to the oesophagus.

If the assist device is fixed in relation to the endoscope in an improper position, problems may occur. For example, a shadow may occur in an image of an object with the treatment instrument advanced through the instrument channel of the assist device. Also, white smear may occur in the image with light improperly reflected by the treatment instrument in an illuminating condition.

Document US6878106 discloses the preamble of claim 1.

### SUMMARY OF THE INVENTION

In view of the foregoing problems, an object of the present invention is to provide an endoscope system in which a nasal type of an endoscope is used for medical treatment and in which an assist device can be positioned suitably, and the assist device for use in combination with the nasal endoscope.

In order to achieve the above and other objects and advantages of this invention, according to a first aspect of the invention there is provided: an endoscope system having an endoscope including a first elongated tube, having a first distal portion, for entry in a first external nostril, an image pickup device for picking up an image of an object through an imaging window formed in a first distal end surface of said first distal portion, a first instrument channel, contained in said first elongated tube, for extending from said first distal portion toward a proximal portion, a handle section connected with said proximal portion, and a steering section for steering said first distal portion at least in an upward or downward direction upon steering operation of a steering mechanism disposed in said handle section, said endoscope system comprising: an assist device including a second elongated tube, having a second distal portion, for entry in a second external nostril, and a second instrument channel, defined in said second elongated tube, for extending from a second distal end surface of said second distal portion in a proximal direction, said second instrument channel having a greater inner diameter than said first instrument channel; a display panel for operating according to an image signal of said image picked up by said image pickup device, and displaying said image in a state of orienting an upper side of steering of said steering section in said upward direction; a retaining device for retaining said second distal portion on said first distal portion removably to orient said first and second distal end surfaces in an equal direction, to cause said display panel to display an image of a medical instrument through said second instrument channel in a partial area lower than a centre point of a display area thereof; wherein said imaging window is positioned eccentrically with reference to a centre point of said first distal end surface; and characterized by further comprising two lighting windows formed in said first distal end surface, for emitting light toward said object, wherein said retaining device holds said assist device, such that said assist device is located at one of two areas defining an outer periphery of said first distal portion, in which said imaging window is not disposed, said two areas being defined by a straight line which is tangential to circumferences of said two lighting windows and which is perpendicular to a central line passing a centre point of said imaging window and said centre point of said first distal end surface.

Preferably, said second elongated tube includes a rigid distal portion, a steering section, and a flexible tube.

Consequently, it is possible in the present invention suitably to position the assist device for use with a nasal type of endoscope for medical treatment.

### BRIEF DESCRIPTION OF THE DRAWINGS

The above objects and advantages of the present invention will become more apparent from the following detailed description when read in connection with the accompanying drawings, in which:
Fig. 1 is a perspective view illustrating one preferred endoscope system including an endoscope and an assist device;
Fig. 2 is a cross section illustrating an elongated tube of the endoscope;
Fig. 3 is a front elevation illustrating a distal end surface of the elongated tube;
Fig. 4 is a vertical section illustrating the inside of a distal portion;
Fig. 5 is a cross section illustrating an elongated tube of the assist device;
Fig. 6 is a vertical section illustrating the inside of a distal portion of the assist device;
Fig. 7 is a front elevation, partially in a section illustrating a first distal end surface and a peripheral part determined for magnetic attraction;
Fig. 8 is a cross section illustrating magnets in the distal portion of the assist device;
Fig. 9 is a perspective view illustrating a secured state of the distal portions of the endoscope and assist device;
Fig. 10 is a front elevation illustrating a specifically preferred peripheral part for magnetic attraction in the secured state of the distal portions;
Fig. 11 is a plan illustrating a displayed image of a forceps device;
Fig. 12. is a block diagram schematically illustrating connection of various elements in the endoscope system;
Fig. 13 is an explanatory view illustrating switching of a flow for air with the air/water supply button;
Fig. 14 is an explanatory view illustrating switching of a flow for water with the air/water supply button;
Fig. 15 is a perspective view illustrating a suction device with a suction connector;
Fig. 16 is a flow chart illustrating endoscopy or treatment by use of the endoscope system;
Fig. 17 is a vertical section illustrating a nasal tract and entry of the endoscope or assist device;
Fig. 18 is an explanatory view illustrating one preferred endoscope system in which a proximal end of the assist device is free from the endoscope;
Figs. 19 and 19A are perspective views illustrating another preferred endoscope system having a snare device for the retention;
Fig. 20 is a perspective view illustrating another endoscope system in which an assist device operates for lighting;
Fig. 21 is a cross section illustrating an elongated tube of the endoscope;
Fig. 22 is a front elevation illustrating a distal end surface of the elongated tube;
Fig. 23 is a vertical section illustrating the inside of a distal portion;
Fig. 24 is a cross section illustrating an elongated tube of the assist device;
Fig. 25 is a vertical section illustrating the inside of a distal portion of the assist device;
Fig. 26 is a front elevation illustrating the inside of the distal portion of the assist device;
Fig. 27 is a perspective view illustrating a secured state of the distal portions of the endoscope and the assist device;
Fig. 28 is a plan illustrating a displayed image of a forceps device;
Fig. 29 is a perspective view illustrating a relationship between an axis of a moving domain of the forceps device and a light flux;
Fig. 30 is a block diagram schematically illustrating circuit elements of the endoscope system;
Fig. 31 is a block diagram schematically illustrating mechanically connected elements in the endoscope system;
Fig. 32 is a perspective view illustrating a light source apparatus;
Fig. 33 is a flow chart illustrating endoscopy and treatment by use of the endoscope system;
Fig. 34 is a front elevation illustrating an assist device settable in one of two mounting surfaces;
Fig. 35 is a front elevation illustrating another assist device with two lighting windows;
Fig. 36 is a vertical section illustrating a distribution characteristic of the two lighting windows;
Fig. 37 is a front elevation illustrating an assist device with plural sets of lighting windows.

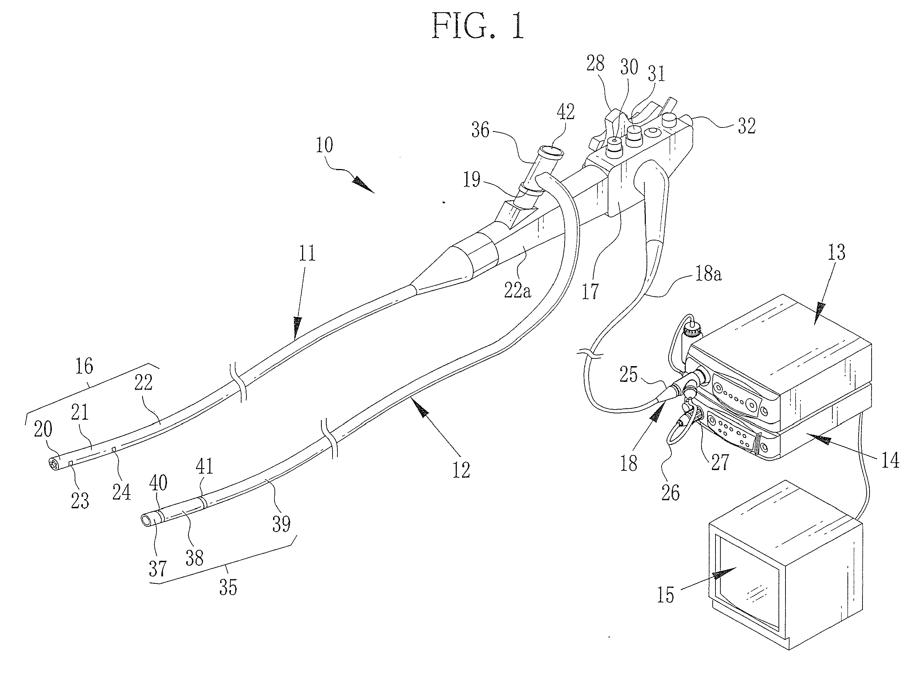

It should be noted that only the endoscope system shown in Figures 1 to 19 falls within the scope of the claims.

### DETAILED DESCRIPTION OF THE PREFERRED EMBODIMENT(S) OF THE PRESENT INVENTION

In Fig. 1, an endoscope system 10 includes a nasal endoscope 11, an assist device 12 or endoscopic catheter, a light source apparatus 13, a processing apparatus 14 and a display panel 15. A first elongated tube 16 or insertion tube is included in the endoscope 11, and used for entry through a first one of nostrils of a patient or person. A grip portion 22a extends from the first elongated tube 16. A handle section 17 is secured to the grip portion 22a. On the handle section 17, a universal connector 18 is formed at an end of a universal cable 18a, and becomes connected with the light source apparatus 13 and the processing apparatus 14. A tube lumen is formed through the first elongated tube 16 of the endoscope 11. An instrument channel is contained in the tube lumen. A first end of the instrument channel constitutes a distal opening in a distal portion of the first elongated tube 16. A second end of the instrument channel constitutes a proximal opening 19 or inlet in the grip portion 22a. Note that the proximal opening 19 may be formed in the handle section 17. A handle is constituted by the grip portion 22a and the handle section 17.

As is well-known in the art, the first elongated tube 16 of the endoscope 11 includes a first distal portion 20 with a head assembly, a steering section 21 and a flexible tube 22. Two annular magnets or magnetic components 23 and 24 are disposed at ends of the steering section 21 for combined use with the assist device 12. Note that the steering section 21 is important additionally to the first distal portion 20 of the first elongated tube 16 of the endoscope 11 of the invention.

In the endoscope 11, the first distal portion 20 includes a rigid tubular structure and relevant devices contained in the tubular structure. The devices include imaging optics, an image pickup device and lighting optics. The universal connector 18 includes a light guide coupler 25 and a video connector 27 or plug. A signal cable 26 extends from the universal connector 18, and has the video connector 27 disposed at its end. The video connector 27 is connected with the processing apparatus 14. The light guide coupler 25 is connected with the light source apparatus 13.

A power source circuit is contained in the processing apparatus 14. Also, an image processor is incorporated in the processing apparatus 14, and encodes the image to a composite signal or RGB component signal by image processing of the image signal from the image pickup device. The light source apparatus 13 contains a lamp, of which light passes through the handle section 17 and light guide optics (fibre bundle) inside the first elongated tube 16. The light is guided from the grip portion 22a to the distal end, and enters the lighting optics.

The flexible tube 22 has flexibility, and extends between the handle section 17 and the steering section 21 with a small diameter. Steering wheels 28 or steering mechanism is disposed on the handle section 17. Steering wires for steering are contained in a tube lumen in the first elongated tube 16, and are moved back and forth by rotating the steering wheels 28 for steering. Thus, a distal end surface of the first distal portion 20 is directed in a desired direction in a body cavity to image an object. The object is illuminated by light emitted by the lighting optics. Reflected light from the object is picked up by an image pickup device through imaging optics, for the display panel 15 to display an image by use of an image processing circuit.

An air/water supply button 30, a suction button 31 and a water jet opening 32 are disposed on the handle section 17 in addition to the steering wheels 28 and the proximal opening 19. To the water jet opening 32, a syringe, water supply device or the like are connected in a removable manner, the syringe containing washing water, drug solution and other fluid for ejection to an object in the body. In a normal state, the water jet opening 32 and the proximal opening 19 are closed tightly by a seal or lid fitted in a removable manner.

The assist device 12 is combined with the endoscope 11, and includes a second elongated tube 35 or insertion tube and an engaging portion 36. The second elongated tube 35 is entered through a second nostril which is different from the first nostril of entry of the first elongated tube 16 of the endoscope 11. The engaging portion 36 is positioned at a proximal end of the second elongated tube 35, and is engaged with the grip portion 22a or the handle section 17 of the endoscope 11 in a removable manner. The second elongated tube 35 of the assist device 12 includes a second distal portion 37, a steering section 38 and a flexible tube 39 in a sequence in a proximal direction.

Annular magnets or magnetic components 40 and 41 are disposed on the steering section 38 of the assist device 12 and arranged in the axial direction of the endoscope. The magnets 40 and 41 magnetically attract the magnets 23 and 24 on the steering section 21 of the endoscope 11 when entered in a range from the internal nostril to the oesophagus. Thus, the steering section 38 of the assist device 12 is bent for steering together with the bend of the steering section 21 of the endoscope 11. A distal end surface of the second distal portion 37 of the assist device 12 is directed equally to the distal end surface of the first distal portion 20 of the endoscope 11.

The second distal portion 37 of the assist device 12 is formed from rigid material. The steering section 38 is a flexible portion bendable together with the steering section 21 of the endoscope 11. The flexible tube 39 is a long flexible portion with a small diameter, and extends between the engaging portion 36 and the steering section 38. The steering section 38 is a portion extending from the second distal portion 37 as an important element in the second elongated tube 35 of the assist device 12 of the invention.

An instrument channel is formed through the second elongated tube 35 of the assist device 12 to extend from the distal end to the engaging portion 36. A first end of the instrument channel constitutes a distal opening in the distal end surface of the assist device 12. A second end of the instrument channel constitutes an additional proximal opening 42 or inlet in the engaging portion 36. A sealing film 42a or channel lid is fitted to close the additional proximal opening 42. A sealing portion of the sealing film 42a has flexibility, includes slits or small pores, and is openable when pushed by a medical instrument. The sealing film 42a partially seals the additional proximal opening 42 and prevents fluid from flowing out of a second instrument channel or working channel 72. The engaging portion 36 is engaged with the proximal opening 19 in the handle section 17 in a removable manner. A short path or bypass line is defined by the engaging portion 36 for connecting the instrument channel of the assist device 12 with the proximal opening 19 of the handle section 17. This enables selection of the instrument channel for use between the endoscope 11 and the assist device 12 by changing the direction of the distal end after inserting the forceps device into the additional proximal opening 42 of the assist device 12.

For advance in a path from the external nostril, internal nostril, and oesophagus to the stomach and duodenum with steerability, the elongated tubes 16 and 35 have a small diameter with flexibility, and have the diameter and length equal between those. For the purpose of endoscopic treatment, the engaging portion 36 of the assist device 12 can be engaged with the handle section 17 either before or after the entry of the elongated tubes 16 and 35 in a patient's body. For the subsequent engaging step, a length of the second elongated tube 35 of the assist device 12 preferably can be greater than a length of the first elongated tube 16 of the endoscope 11 in view of easy entry. Note that there may be a patient in which the first elongated tube 16 of the endoscope 11 cannot be entered through a first one of his or her nostrils according to a test. Accordingly, a diameter of the second elongated tube 35 of the assist device 12 is preferably smaller than a diameter of the first elongated tube 16 of the endoscope 11.

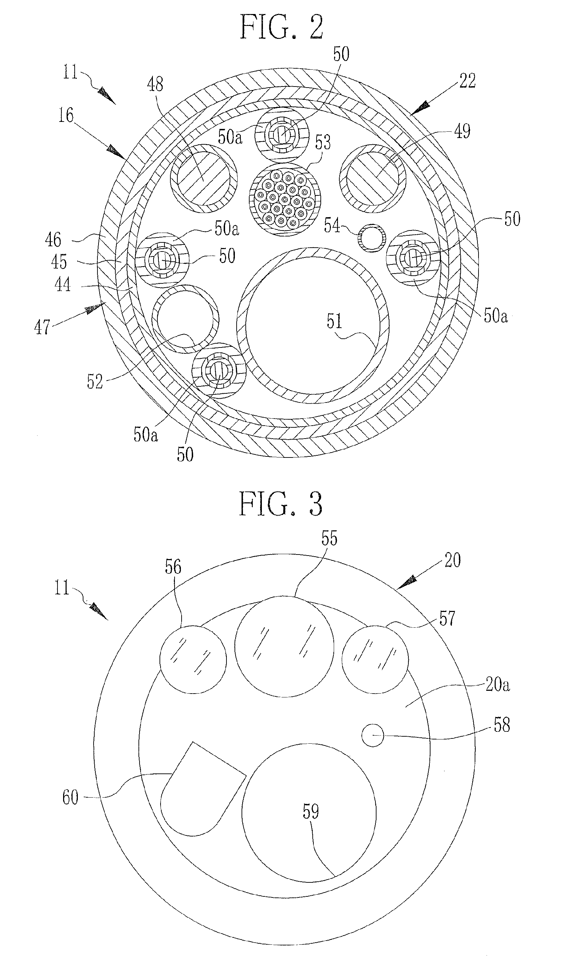

In Fig. 2, a flexible structure 47 constitutes the flexible tube 22 of the endoscope 11. The flexible structure 47 is a three layer structure and includes a helical coil 44, a mesh material 45 and a covering layer 46. The helical coil 44 is flexible, and defines a tube lumen where constituent components are contained. The mesh material 45 is disposed around the helical coil 44. The covering layer 46 wraps the mesh material 45 in an overlaid manner and is reinforced by the mesh material 45.

A plurality of constituent components are loosely inserted in the flexible tube 22 of the endoscope 11, including light guide optics 48 and 49, steering wires 50, an instrument channel 51, an air/water supply channel 52, a multi-core cable 53, and a water jet channel 54. The multi-core cable 53 is a cable for transmitting a signal from a signal processor to drive the image pickup device, and for transmitting an image signal from the image pickup device to the image processor. The multi-core cable 53 includes plural signal lines and a protecting layer for wrapping the signal lines. The steering wires 50 are four wires contained in the flexible tube 22, extend toward the steering section 21 to contact the periphery of two pulleys in a turnable manner for steering vertically and horizontally upon rotation of the steering wheels 28. There is a winding 50a with tightly wound strands, in which each of the steering wires 50 is inserted.

A first distal end surface 20a is positioned at an end of the first distal portion 20 of the endoscope 11. In Fig. 3, various elements are disposed on the first distal end surface 20a, including an imaging window 55, two lighting windows 56 and 57, a water jet nozzle 58, a distal opening 59 or outlet, and an air/water supply nozzle 60. The imaging window 55 is constituted partially by imaging optics for receiving object light of an object in the body. The lighting windows 56 and 57 are disposed beside the imaging window 55, and apply light from the light source apparatus 13 to the object in the body through the light guide optics 48 and 49.

The distal opening 59 of the endoscope 11 is formed to communicate from the instrument channel 51 with the proximal opening 19 at the handle section 17. The air/water supply nozzle 60 operates in response to operation of the air/water supply button 30 at the handle section 17, draws air or water to an object in the patient's body, and ejects air or water to the imaging window 55 for cleaning. The water jet nozzle 58 ejects fluid to the object from a syringe connected with the water jet opening 32 in a removable manner, the fluid being such as washing water, drug solution or the like.

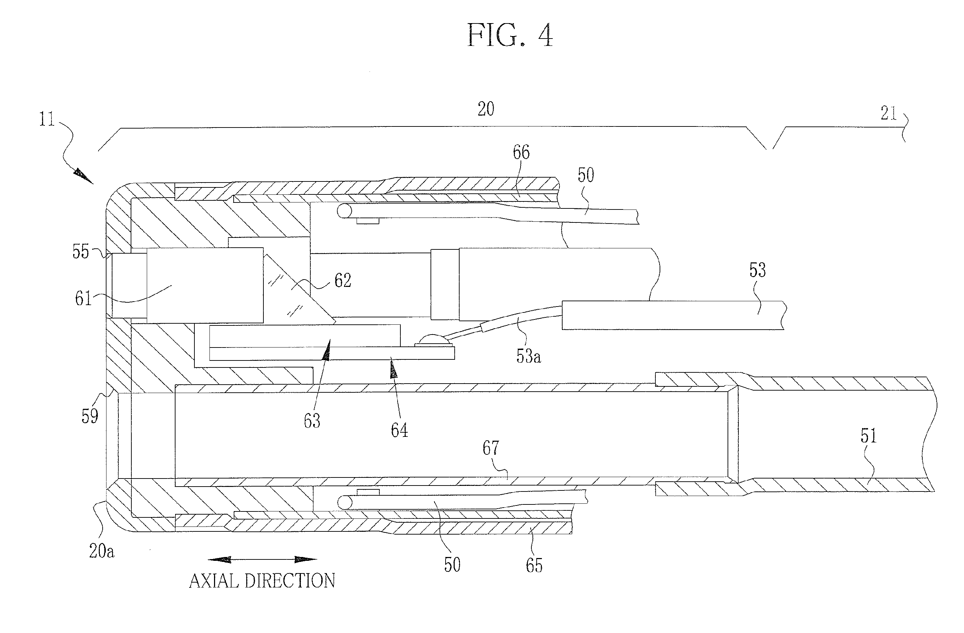

In Fig. 4, an objective optical system or imaging optics 61 are disposed to appear partially in the imaging window 55. Light from the lighting windows 56 and 57 is reflected by the object in the body, and enters the imaging optics 61. A prism 62 receives entry of the object light from the imaging optics 61, and refracts the light. An image pickup device 63 has a light receiving surface where the refracted object light is focused in an originally erected orientation. The image pickup device 63 is kept fixed so that an upper direction of steering of the steering section 21 is set equal to an upper side of an image on the display panel 15. A circuit board 64 is connected with the image pickup device 63. Plural signal lines 53a in the multi-core cable 53 are connected with the circuit board 64.

A rubber layer 65 for steering is overlaid on an intermediate layer of the first distal portion 20 and the steering section 21 of the endoscope 11. A connection ring 66 is disposed inside the rubber layer 65, and receives engagement of a distal end of the steering wires 50. A plurality of chain links (not shown) are arranged and interconnected with pins in a direction toward the proximal side. The pins are oriented alternately in the vertical direction and then in the horizontal direction as pivotal axes of the chain links. The steering wires 50 are engaged with the inside of the chain links in the slideable manner. The chain links for steering make horizontal and vertical curves upon moving back or forth of the steering wires 50.

The instrument channel 51 is disposed in the steering section 21 of the endoscope 11 and extends through the flexible tube 22. The instrument channel 51 is formed from flexible synthetic resin. A rigid pipe 67 is connected with a distal end of the instrument channel 51 and disposed inside the first distal portion 20. A distal end of the rigid pipe 67 is positioned in the distal opening 59.

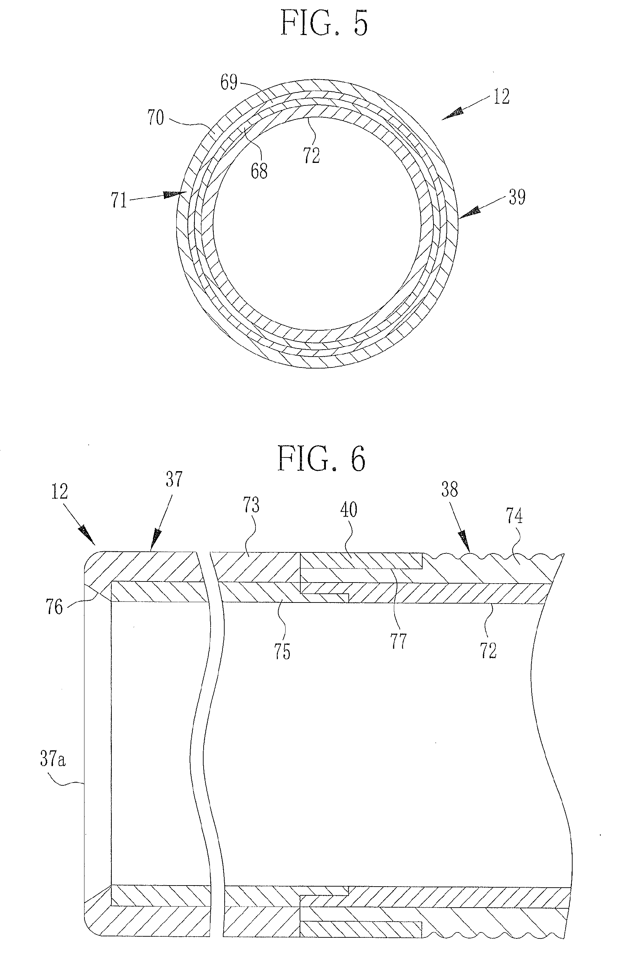

There is a flexible structure 71 constituting the flexible tube 39 of the assist device 12. In Fig. 5, the flexible structure 71 is a three layer structure including a helical coil 68, a mesh material 69 and a covering layer 70 in a manner similar to the flexible tube 22 of the endoscope 11. The helical coil 68 is disposed around the working channel 72, is flexible and contains the working channel 72. The mesh material 69 is disposed around the helical coil 68. The covering layer 70 wraps the mesh material 69 in an overlaid manner and is reinforced by the mesh material 69.

The flexible structure 71 of the assist device 12 is formed from flexible synthetic resin. A bore of the working channel 72 is at least 70 % as great as an outer diameter of the second elongated tube 35 of the assist device 12, and at least two times as great as a bore of the instrument channel 51 of the endoscope 11. Thus, a medical instrument of a large size can be inserted in the working channel 72 of the assist device 12.

In Fig. 6, an end ring 73 is an outer covering part of the second distal portion 37 of the assist device 12. A distal opening 76 or outlet is formed in a second distal end surface 37a of the second distal portion 37. A support sleeve 75 or rigid pipe is connected with the distal opening 76. The working channel 72 extends to communicate with the support sleeve 75. An inner bore in the assist device 12 is equal between the distal opening 76, the support sleeve 75 and the working channel 72.

An outer layer 74 is positioned externally in the steering section 38 of the assist device 12, and bendable together with the steering section 21 of the endoscope 11. The outer layer 74 of the steering section 21 is structurally the same as the flexible tube 39, but is slightly shiftable to expand and contract in the axial direction to prevent offsetting of the second distal end surface 37a of the second distal portion 37 from the first distal end surface 20a of the endoscope 11 when bent internally or externally upon bending of the steering section 38 of the endoscope 11.

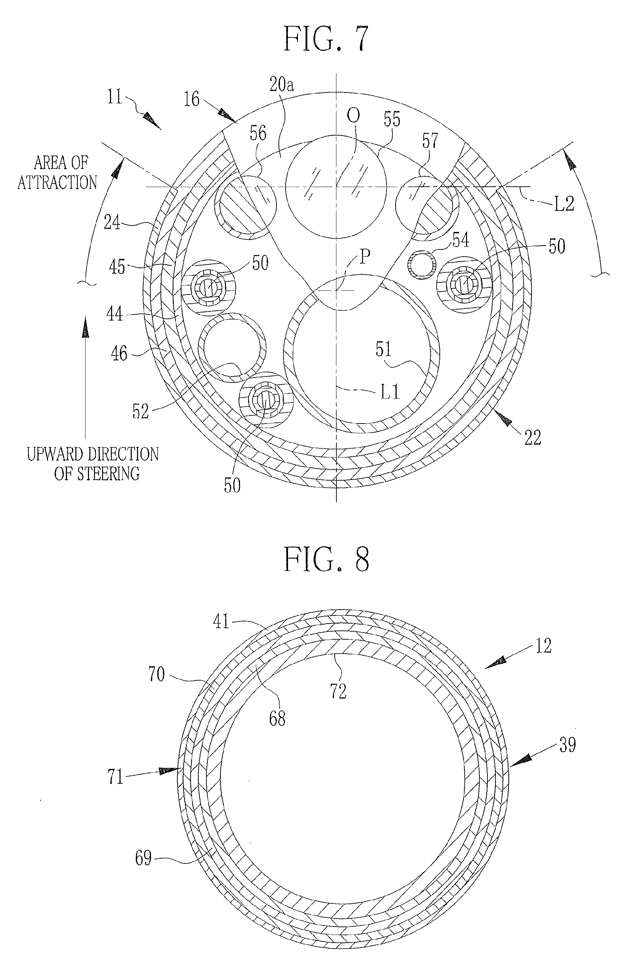

In Fig. 7, the two magnets on the peripheral surface of the first elongated tube 16 of the endoscope 11 has a C-shape as viewed in a cross section, namely, an arcuate shape by partially cutting a ring. A centre O of the imaging window 55 is positioned on a line L1 passing the centre P of the first distal end surface 20a and higher than the centre P of the first distal end surface 20a when the first distal end surface 20a of the endoscope 11 is viewed in a rotational position of directing an upper portion of the steering section 21 of the steering in an upward direction. An area of magnetic attraction of the magnets 23 and 24 is a range of securing a distal portion of the second elongated tube 35 of the assist device 12 lower than a horizontal line L2 passing the centre O of the imaging window 55 when the first distal end surface 20a of the endoscope 11 is viewed in the rotational position of directing the upper portion of the steering section 21 of the steering in the upward direction. In Fig. 8, the magnets 40 and 41 on the assist device 12 have a ring shape as viewed in a cross section.

Cut-outs 77 and 78 are formed in the outer layer 74. The magnets 40 and 41 are arranged on the steering section 38 in the axial direction of the assist device 12, fitted in respectively the cut-outs 77 and 78, and set to have a surface flush with the outer layer 74. Cut-outs 79 and 80 are formed in the outer layer 74. The magnets 23 and 24 are arranged on the steering section 21 in the axial direction of the endoscope 11, fitted in respectively the cut-outs 79 and 80, and set to have a surface flush with the outer layer 74. A predetermined distance between the magnets 40 and 41 is equal to that between the magnets 23 and 24. The steering sections 21 and 38 of the endoscope 11 and the assist device 12 can be retained on one another to orient the distal end surfaces 20a and 37a by the magnetic attraction at the two points with the predetermined distance.

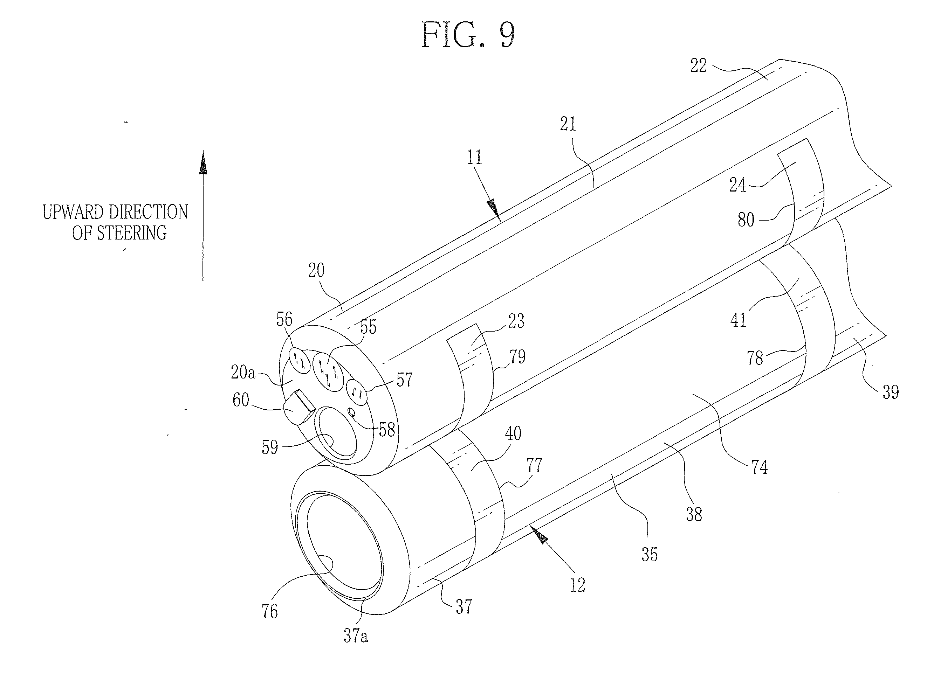

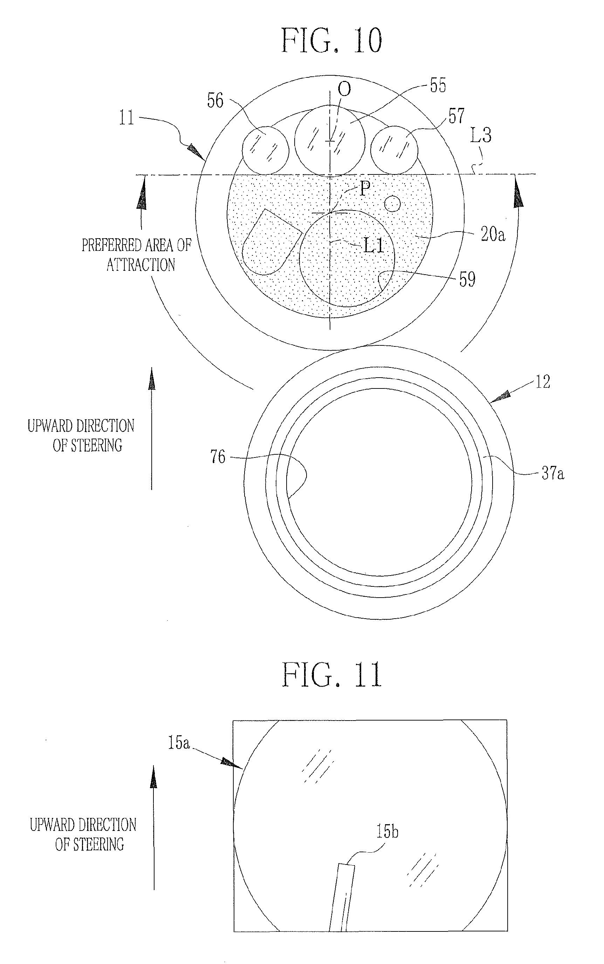

In Fig. 9, a distal portion of the second elongated tube 35 of the assist device 12 is fixedly secured to a distal portion of the first elongated tube 16 of the endoscope 11 for the second distal end surface 37a of the assist device 12 to contact a lower surface of the first elongated tube 16 under a horizontal line L2 passing the centre O of the imaging window 55. As illustrated in Fig. 10, a range of securing the distal end of the assist device 12 can be preferably defined to dispose the magnets 23 and 24 in a peripheral part which is defined by a line L3 being perpendicular to a line L1 passing the window centre point and the centre P of the distal end surface, the line L3 being tangential to the outside of the lighting windows 56 and 57, the peripheral part being contact with a dotted area which is outside an area containing the lighting windows 56 and 57.

In Fig. 11, the display panel 15 displays an image of an object in an orientation of setting the upper side of the steering section 21 upwards. An image 15b or shadow of the forceps device or medical instrument is displayed in an area under a centre of a display frame 15a when the elongated tubes 16 and 35 are fixedly set in the positions described with Figs. 9 and 10.

The retaining device of the invention is constituted by the magnets 23 and 24 and the magnets 40 and 41. Alternatively, plural magnets may be attached to a ring shaped belt, which can be mounted on the first elongated tube 16 of the endoscope 11. For this structure, a positioning portion is formed with the mounting surface for the magnets to lie in the area of the attraction depicted in Fig. 7.

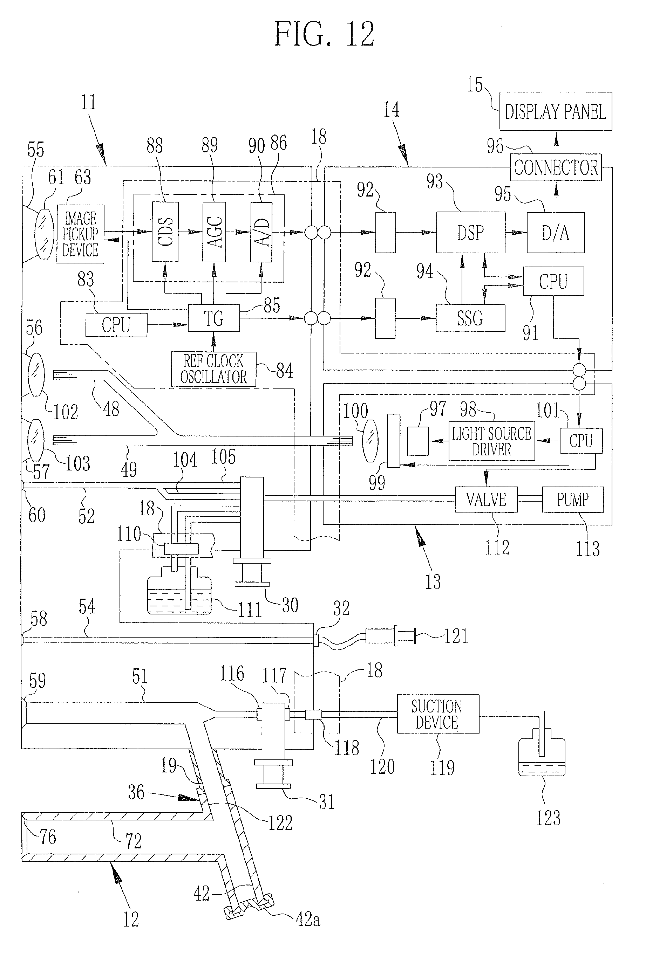

The endoscope 11 has the image pickup device 63 of Fig. 12 in the first distal portion 20. Also, the endoscope 11 includes a CPU 83, a reference clock oscillator 84, a TG (timing generator) 85, and an AFE (analogue front end circuit) 86 or AFE processor, all contained in the universal connector 18.

Examples of the image pickup device 63 are a CCD or CMOS image sensor and the like, to pick up an object image focused by the imaging optics 61. A colour filter is disposed on a light receiving surface, and includes plural colour segments, for example, a colour filter of primary colours of the Bayer array.

The CPU 83 controls various elements in the endoscope 11. The TG 85 creates drive pulses (vertical and horizontal) according to the reference clock signal from the reference clock oscillator 84, creates a sync pulse for the AFE 86, and supplies the image pickup device 63 and the AFE 86 with respectively the drive pulses and the sync pulse. The image pickup device 63 responds to a drive pulse input by the TG 85, picks up an image, and outputs an image signal to the AFE 86.

The AFE 86 includes a CDS (correlated double sampling circuit) 88, an AGC (automatic gain control) circuit 89 and an A/D converter 90. The CDS 88 processes the image signal from the image pickup device 63 in the correlated double sampling, and removes reset noise and amplification noise generated in the image pickup device 63 from the image signal. The AGC circuit 89 adjusts the gain of the image signal from the CDS 88 after the noise removal. The A/D converter 90 converts the amplified image signal from the AGC circuit 89 into a digital signal of a predetermined number of bits, and sends the digital signal to the processing apparatus 14 through the universal connector 18.

The TG 85 inputs the horizontal and vertical sync signals and clock signal to the processing apparatus 14 through the universal connector 18 according to the image signal from the AFE 86.

The processing apparatus 14 includes a CPU 91, an isolator 92, a digital signal processor (DSP) 93, a sync signal generator (SSG) 94 and a D/A converter 95.

The CPU 91 controls the processing apparatus 14 and the light source apparatus 13. The isolator 92 operates for isolating the endoscope 11 from the processing apparatus 14. The digital signal processor 93 creates a video signal by signal processing of the image signal.

The sync signal generator 94 is supplied by the isolator 92 with a horizontal drive pulse, vertical drive pulse and clock pulse which have been output by the TG 85 of the endoscope 11. The sync signal generator 94 corrects a phase difference between the horizontal drive pulse, vertical drive pulse and clock pulse. The corrected horizontal drive pulse, vertical drive pulse and clock pulse are generated and input to the digital signal processor 93.

The image signal from the AFE 86 of the endoscope 11 is supplied through the isolator 92 to the digital signal processor 93. The digital signal processor 93 processes the image signal for various functions such as colour separation, colour interpolation, gain correction, white balance adjustment, gamma correction, image enhancement, and the like, and produces a video signal of the Y/C format including luminance (Y) and chrominance (C). The video signal is output to the D/A converter 95. The D/A converter 95 converts the video signal into an analogue video signal of the NTSC format. There is a connector 96 or plug through which the D/A converter 95 outputs the analogue video signal to the display panel 15.

The light source apparatus 13 includes an illuminator 97 or lamp, a light source driver 98, an aperture stop device 99, a condensing lens 100 and an illumination CPU 101. Examples of the illuminator 97 are a xenon lamp, halogen lamp and other white light sources. The light source driver 98 drives the illuminator 97. The aperture stop device 99 is disposed between the illuminator 97 and entrance ends of the light guide optics 48 and 49, and adjusts a light amount of the light incident upon the light guide optics 48 and 49. The condensing lens 100 condenses light from the aperture stop device 99, and directs the light to the entrance ends of the light guide optics 48 and 49. The illumination CPU 101 communicates with the CPU 91 of the processing apparatus 14, and controls the light source driver 98 and the aperture stop device 99. Light emitted by the illuminator 97 passes through the aperture stop device 99 and the condensing lens 100, and becomes incident upon the entrance ends of the light guide optics 48 and 49. Lighting lenses 102 and 103 are disposed to receive the light exited from the exit ends of the light guide optics 48 and 49, and pass the light to travel toward the object in the body through the lighting windows 56 and 57.

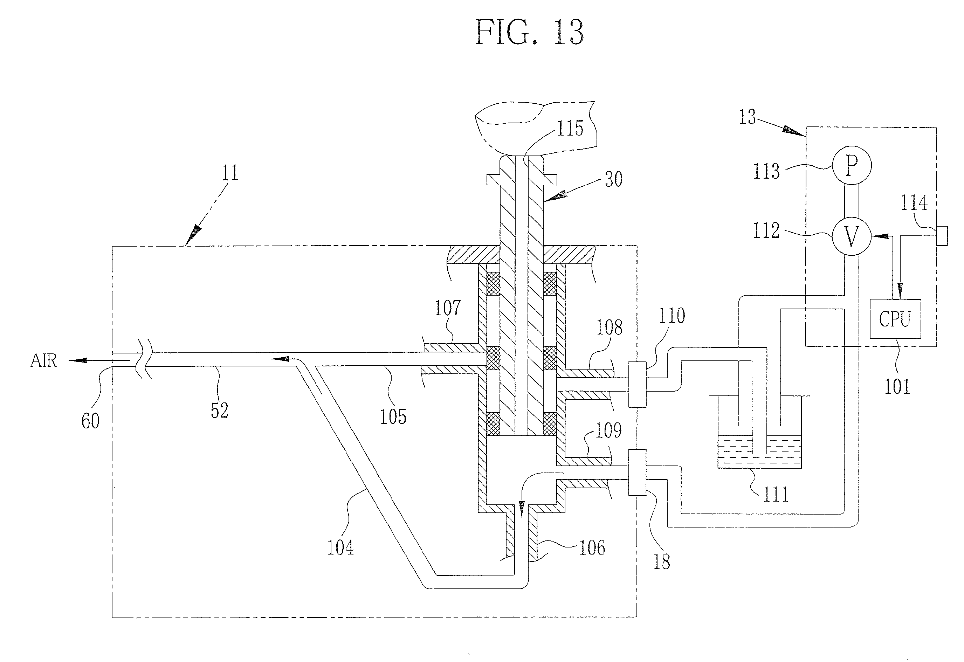

A proximal end of the air/water supply channel 52 in connection with the air/water supply nozzle 60 in the endoscope 11 has an air supply channel 104 and a water supply channel 105 provided by branching. The air supply channel 104 and the water supply channel 105 are respectively connected with the air/water supply button 30. In Fig. 13, the air/water supply button 30 operates for switching the flow, and includes a first outlet orifice 106 for air, a second outlet orifice 107 for water, a second inlet orifice 108 for water, and a first inlet orifice 109 for air. The water supply channel 105 is connected with the second outlet orifice 107. The air supply channel 104 is connected with the first outlet orifice 106. A water supply conduit 110 is formed to extend through the universal connector 18. A water supply tank 111 is connected with the second inlet orifice 108 by the water supply conduit 110. A valve 112 and an air supply pump 113 are incorporated in the light source apparatus 13 and are connected with the first inlet orifice 109 by the universal connector 18.

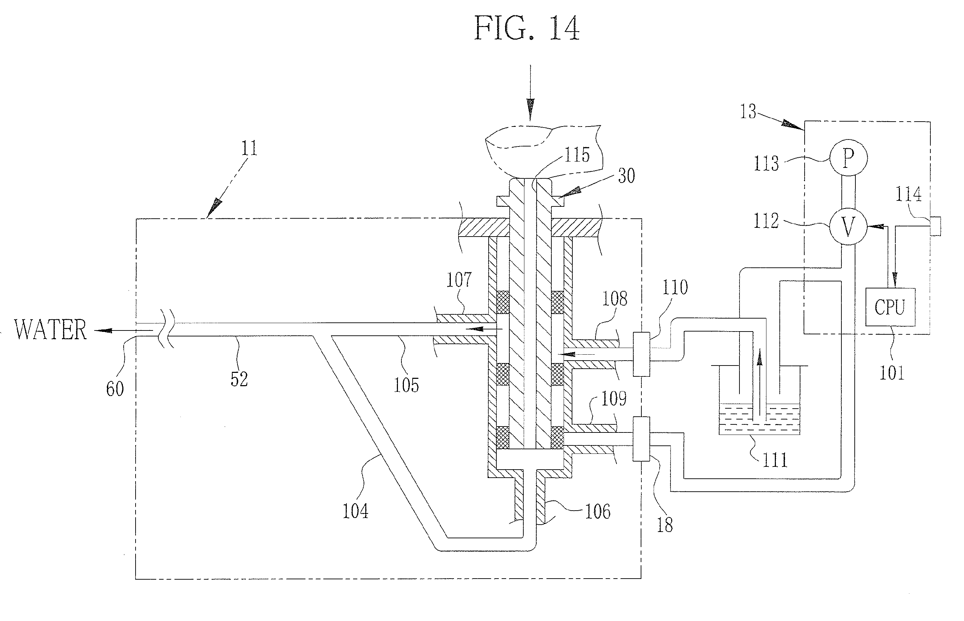

An air supply button 114 is disposed to appear externally on the light source apparatus 13, and operable for setting pressure of an air flow. Information of the pressure set by the air supply button 114 is sent to the illumination CPU 101, which adjusts the valve 112 according to the pressure information. There is a vent 115 formed through the air/water supply button 30, for drawing out air as the air/water supply button 30 is driven constantly. In Fig. 14, the structure is depicted more specifically. When the air/water supply button 30 is depressed, the first inlet orifice 109 is closed to draw air into the water supply tank 111. The air ejects water from the endoscope 11. The water flows through the second inlet orifice 108, the second outlet orifice 107 and the air/water supply channel 52, and is ejected through the air/water supply nozzle 60.

In Fig. 12, the water jet channel 54 contained in the first elongated tube 16 of the endoscope 11 has a first end connected with the water jet nozzle 58 and a second end connected with the water jet opening 32 in the handle section 17. A syringe 121 or a tube in connection with the syringe 121 is connected with the water jet opening 32. When a plunger in the syringe 121 is slid in an axial direction, fluid in the syringe 121 is drawn to flow from the water jet channel 54 toward a lesion through the water jet nozzle 58.

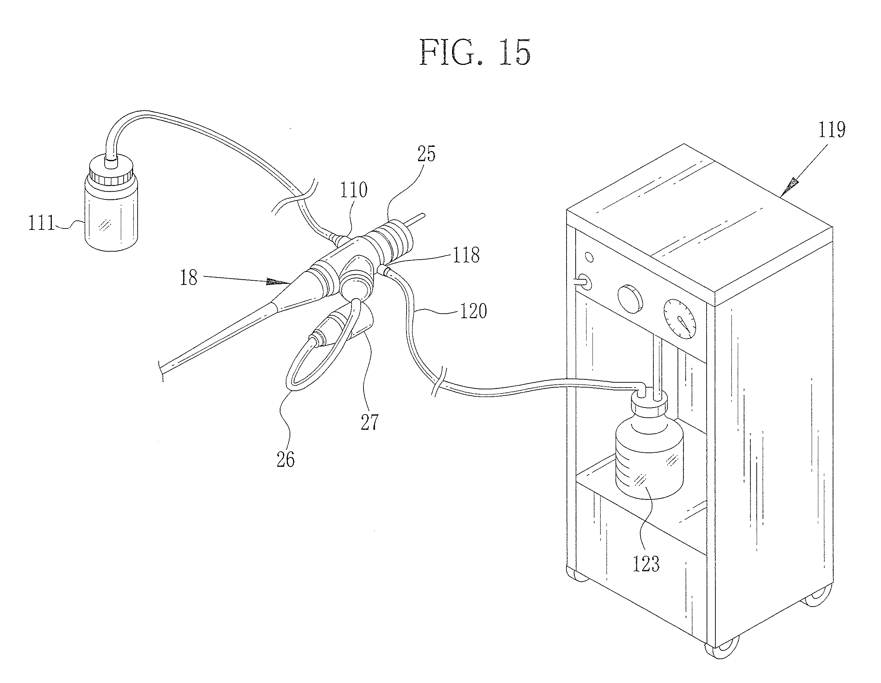

A proximal end of the instrument channel 51 of the endoscope 11 has branches extending to the proximal opening 19 and to the suction button 31 of the handle section 17. In the suction button 31, a port 116 is connected with the instrument channel 51. A port 117 is connected with a suction connector 118 provided in the universal connector 18. Specifically in Fig. 15, a tube 120 extends between the suction connector 118 and a suction device 119 for connection. When the suction button 31 is depressed, the port 117 comes in register with the port 116. The suction device 119 sucks fluid through the distal opening 59 from the instrument channel 51 of the endoscope 11, for example mucus, blood or the like. A suction tank 123 is installed for collecting the fluid.

In Fig. 12, the engaging portion 36 of the assist device 12 is engaged with the proximal opening 19 of the endoscope 11. The engaging portion 36 includes a short tube 122 or bypass line for connecting the proximal opening 19 of the endoscope 11 with the working channel 72, and an engaging claw (not shown) which is engageable with the proximal opening 19 of the endoscope 11. When the engaging portion 36 is connected with the proximal opening 19, the instrument channel 51 of the endoscope 11 becomes connected with the working channel 72 of the assist device 12. The suction button 31 of the handle section 17 is depressed. As the assist device 12 is combined for use, the suction device 119 can suck and remove fluid rapidly through the distal opening 59 and the distal opening 76 from the instrument channel 51 and the working channel 72 of the endoscope 11 and the assist device 12 or endoscopic catheter, the fluid being such as mucus, blood or the like.

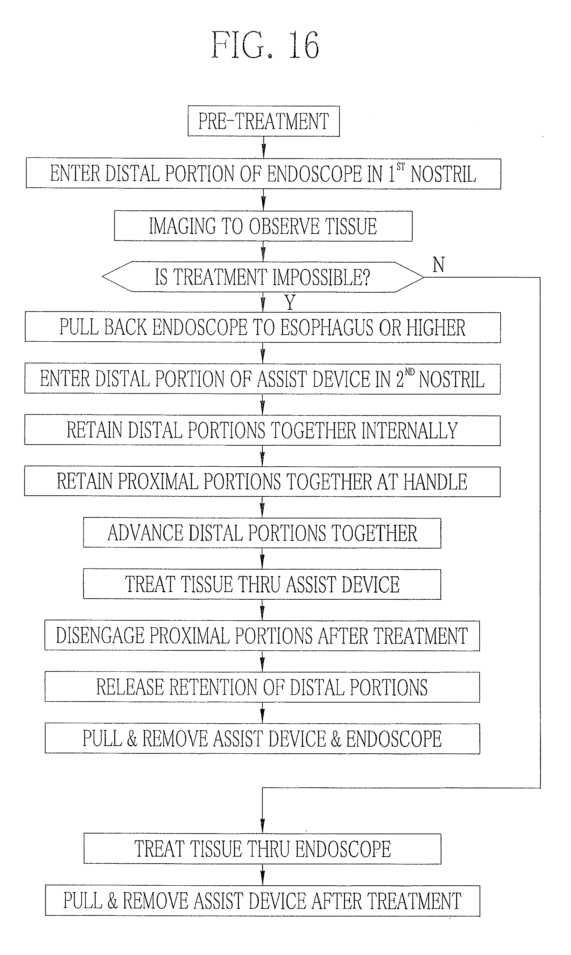

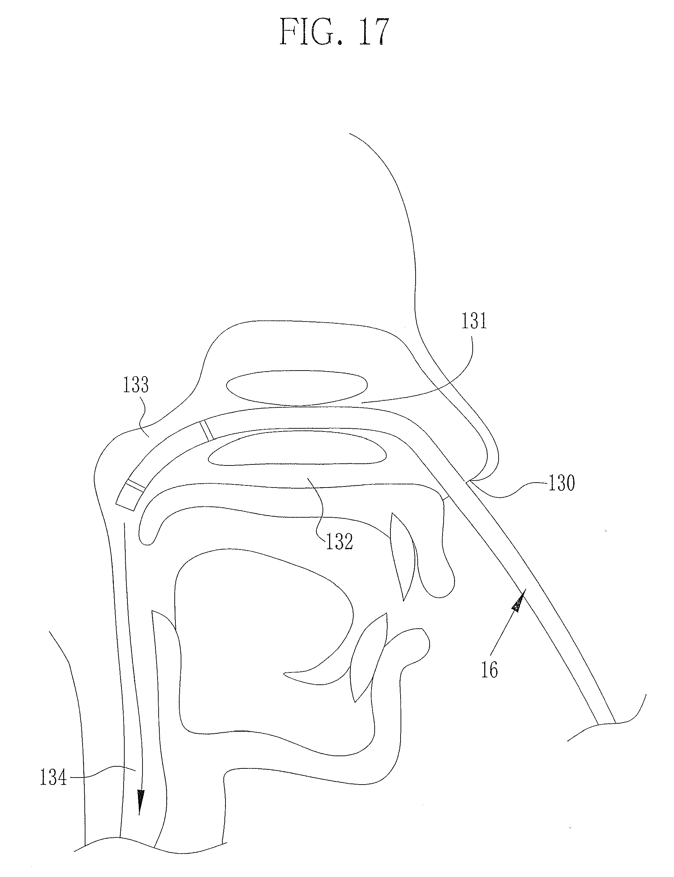

The operation of the above construction is described by referring to Fig. 16. In the nasal endoscopy, a middle or lower nasal tract is anesthetized through a nasal cavity inside a first one of the external nostrils for entry of the first elongated tube 16 of the endoscope 11. A test of the entry is conducted for a doctor to determine one nostril where the first elongated tube 16 can pass for endoscopy. If the first nostril is found too narrow, a second one of the nostrils is used. A nasal tract is anesthetized inside both of the external nostrils. The pre-treatment is carried out while the patient is in the sitting position or supine position. Then the first elongated tube 16 is entered in one external nostril while the patient is in the supine position or left lateral position. In Fig. 17, a path of the entry is illustrated, and extends from one external nostril 130 to either one of a middle nasal tract 131 and a lower nasal tract 132, then to an internal nostril 133 and an oesophagus 134, which extends to a stomach.

If no treatment is required as a result of observing the duodenum, stomach or the like, then the first elongated tube 16 of the endoscope 11 is pulled away from the body. If a lesion is found and if treatment is possible by use of the instrument channel 51 of the endoscope 11, then a medical instrument is entered through the instrument channel 51 to carry out the treatment, for example, a small snare instrument, forceps or the like.

If it is impossible to treat the lesion by use of the instrument channel 51 of the endoscope 11, the assist device 12 is used in combination. To this end, a nasal tract in connection with the second nostril is anesthetized for the purpose of entering the second elongated tube 35 of the assist device 12 in the second nostril. Then the first elongated tube 16 of the endoscope 11 is sufficiently moved back to locate its distal end in a range from the internal nostril 133 to the oesophagus 134 for the purpose of simultaneous entry of the elongated tubes 16 and 35 of the endoscope 11 and the assist device 12 in a retained state. After this, the second elongated tube 35 of the assist device 12 is entered in the second nostril, and moved through the middle nasal tract 131 or the lower nasal tract 132 to a range from the internal nostril 133 to the oesophagus 134. The elongated tubes 16 and 35 are shifted relative to one another in order to position the distal end surfaces 20a and 37a equally in the elongated tubes 16 and 35, so that the magnet 23 of the endoscope 11 attracts the magnet 40 of the assist device 12 and that the magnet 24 of the endoscope 11 attracts the magnet 41 of the assist device 12. This attraction keeps the distal portion of the second elongated tube 35 of the assist device 12 positioned on the distal portion of the first elongated tube 16 of the endoscope 11 in a manner of contact of the second distal end surface 37a of the assist device 12 with the attraction area described with Figs. 7 and 10. Thus, the steering sections 21 and 38 tightly contact one another to direct the distal end surfaces 20a and 37a in the equal direction.

Then the engaging portion 36 of the assist device 12 is engaged with the proximal opening 19 of the endoscope 11. After the engagement, the elongated tubes 16 and 35 are entered gradually in the body. A doctor observes an image on the display panel 15, and rotates the steering wheels 28 to enter the tubes by steering the steering section 21 of the endoscope 11. The steering section 38 of the assist device 12 is bent together with the steering section 21 of the endoscope 11 because stuck on the steering section 21 of the endoscope 11 by magnetic attraction between the magnets 23 and 24 and the magnets 40 and 41. The second elongated tube 35 can advance by following the entry of the first elongated tube 16 of the endoscope 11. It is possible to enter the second elongated tube 35 of the assist device 12 only by operation of handling the first elongated tube 16 of the endoscope 11 for entry.

Then a lesion requiring treatment is displayed on the display panel 15. Then a snare instrument, biopsy forceps device or other instrument is inserted through the additional proximal opening 42 of the engaging portion 36 into the second instrument channel or working channel 72 of the assist device 12. A distal portion of the instrument is moved out of the distal opening 76 of the assist device 12 to carry out the treatment, for example, a pair of biopsy jaws, snare loop, and the like.

The biopsy forceps device as an example of treatment instrument includes a pair of biopsy jaws, a control wire and a flexible sheath. The biopsy jaws are jaw cups secured at a distal end of the control wire in an openable manner. The control wire is inserted through the sheath. A proximal end of the control wire is moved back and forth in the axial direction on the side outside the proximal opening, so as to move the biopsy jaws back and forth through the distal end of the sheath. The biopsy jaws are driven for opening and closing. The biopsy forceps device is mainly used for the purpose of capturing tissue. A required outer diameter of an example of forceps channel adapted to the biopsy forceps device is equal to or more than 2.8 mm.

In a preferred example of snare instrument, the snare loop produced from resilient wire is set movable into and out of an end portion of a sheath in response to manual operation on the sheath. The snare loop, when pulled back in the sheath, is resiliently deformed in a closed state, and when advanced from the sheath, becomes developed in the widely looped shape. For polypectomy with the snare instrument, at first the snare loop is wound on a root portion of the polyp sufficiently tightly. Then a current of high frequency is drawn in the snare loop to burn tissue contacting the snare loop for simultaneous cutting and coagulation. In general, a required outer diameter of the forceps channel suitable for the snare instrument is equal to or more than 2.8 mm.

Through the working channel 72 of the assist device 12, the snare instrument, biopsy forceps device or other instrument is used for biopsy of tissue, or for treatment, for example, removal of foreign material, haemostasis, removal of a tumour, destruction of biliary stones or the like.

The lighting windows 56 and 57 in the first distal end surface 20a of the endoscope 11 are so disposed that the imaging window 55 is between those. See Fig. 3. The second distal end surface 37a of the assist device 12 is located fixedly lower than the imaging window 55. See Fig. 7. Also, the second distal end surface 37a is located fixedly on the periphery associated with an area (dotted in Fig. 10) opposite to the area of the lighting windows 56 and 57. Therefore, the image 15b of the forceps device inserted in the working channel 72 of the assist device 12 is displayed under the centre of the display frame 15a of the display panel 15. This prevents occurrence of a shadow on the object due to blocking light with the forceps device, and occurrence of white defects in an image with light reflected by the forceps device.

In order to suck fluid such as mucus, blood or the like, the suction button 31 of the handle section 17 can be depressed to suck the fluid not only through the distal opening 59 of the endoscope 11 but also through the distal opening 76 of the assist device 12. So the fluid can be sucked rapidly. An amount of the suction can be high, because of simultaneous operation through both of the distal openings 59 and 76.

After the treatment is finished, the medical instrument such as biopsy forceps device and snare instrument is removed from the additional proximal opening 42 of the assist device 12. Then the second elongated tube 35 and the first elongated tube 16 of are removed from the body at a sufficient slow speed. The distal portions of the elongated tubes 16 and 35 are released from their retention before their passage from the internal nostrils to the oesophagus. The magnets 40 and 41 can be released from magnetic attraction on the magnets 23 and 24 by shifting one of the elongated tubes 16 and 35 of the endoscope 11 and the assist device 12 from one another in the axial direction. After the release, the elongated tubes 16 and 35 are removed in the order of the assist device 12 and then the endoscope 11. Finally, the engaging portion 36 of the assist device 12 is removed from the proximal opening 19 of the handle section 17.

Note that there is a situation in which impossibility of the treatment by use of the instrument channel 51 of the endoscope 11 has been found initially. For this situation, the assist device 12 is used as a fixed process. Connection of the engaging portion 36 of the assist device 12 to the handle section 17 may be carried out before entry of the second elongated tube 35 of the assist device 12, or after the retention between the elongated tubes 16 and 35.

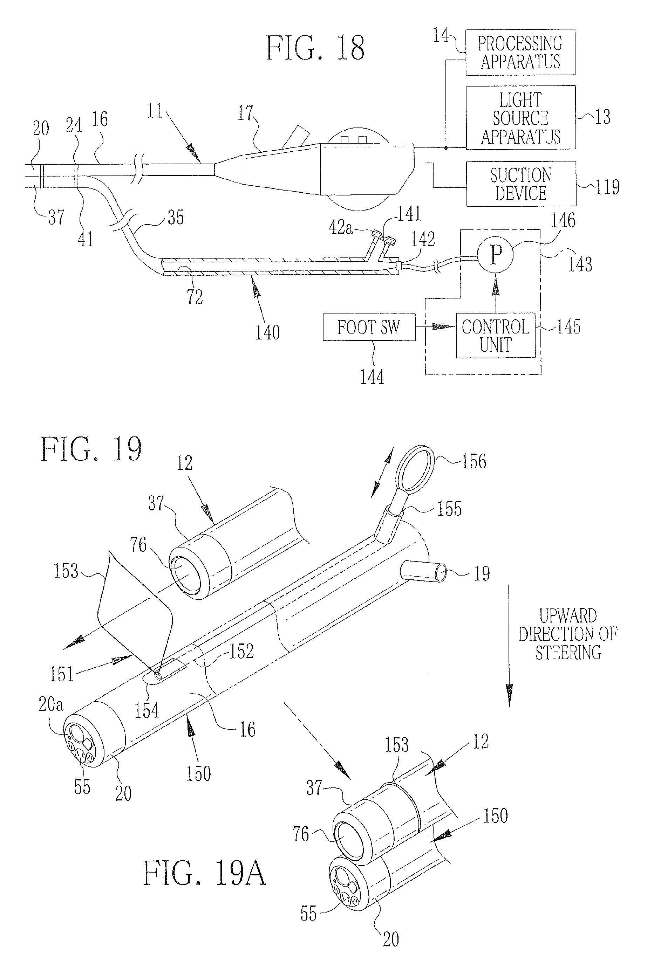

In the above embodiment, the engaging portion 36 is connected with the handle section 17. However, proximal portions of the endoscope 11 and the assist device 12 may have structures without engageable parts. In Fig. 18, another preferred assist device 140 or endoscopic catheter is illustrated. There are a proximal opening 141 or inlet and a suction connector 142 provided by branching at a proximal end of the second instrument channel or working channel 72. A suction device 143 is connectable to the suction connector 142. The suction device 143 includes a control unit 145 and a pump 146, and is structured in a manner specialized for the assist device 140. It is preferable to connect a foot switch 144 to the control unit 145, and to operate the assist device 12 for suction by depressing the foot switch 144 with a foot of a doctor or operator. This is effective in simplifying his or her manual operation when the endoscope 11 is used in combination. In the present embodiment, the suction is possible discretely through the instrument channel 51 of the endoscope 11 and the working channel 72 of the assist device 12. For example, the working channel 72 of the assist device 12 can be used for retrieving relatively large pieces of tissue. The instrument channel 51 of the endoscope 11 can be used for retrieving relatively small pieces of tissue.

In the above embodiments, the magnets 23, 24, 40 and 41 are permanent magnets. However, it is possible in the invention to dispose the electromagnet on a first one of the elongated tubes and the ferromagnetic component on a second one of the elongated tubes. A switching portion for turning on and off the electromagnet may be disposed on the first elongated tube. An electric current flows in the electromagnet upon operation of the switching portion to attract the ferromagnetic component. In a structure of the endoscope 11 having the electromagnet, a current can be drawn through the universal convector 18 from the light source apparatus 13 of the processing apparatus 14. In contrast, in a structure of the assist device 12 having the electromagnet, a terminal can be positioned on a proximal end of the assist device 12 for being supplied with the current. The terminal can be connected with the light source apparatus 13 or the processing apparatus 14. Alternatively, a separate power source can be prepared and can be connected with the terminal of the assist device 12.

In Figs. 19 and 19A, one preferred nasal endoscope 150 having a retaining device 151 with a pull strip is illustrated. A retention lumen 152 is formed through the first elongated tube 16 of the endoscope 150. The retaining device 151 of a snare form is inserted in the retention lumen 152. A retention loop 153 is an end portion of the retaining device 151, and captures and retains the second elongated tube 35 of the assist device 12. This retaining device retains the elongated tubes 16 and 35 to direct the distal end surfaces in the same direction. A lumen outlet 154 is a first end of the retention lumen 152 and is formed at a distal end of the endoscope 150. A lumen inlet 155 is a second end of the retention lumen 152 and is formed in the handle section. The retaining device 151 is constituted by a sheath and a resilient pull strip of wire contained in the sheath in a slideable manner. The retention loop 153 is formed by bending the wire and shiftable between its open and closed states. A pull portion 156 or handle is a proximal end portion of the pull strip of the retaining device 151, and extends outside the lumen inlet 155.

When the pull portion 156 is moved back or forth relative to the sheath, the retention loop 153 of the retaining device 151 is resiliently deformed in a state pulled in the sheath, but becomes developed widely in a looped form in a state shifted in the axial direction. Before entry of the endoscope 150 in the patient's nostril, the retention loop 153 is initially closed. To retain the distal portions on one another, the retention loop 153 spreads in the looped form to receive insertion of the distal portion of the second elongated tube 35 of the assist device 12. Then the pull portion 156 is pulled back to tighten the retention loop 153. Thus, the distal portion of the assist device 12 can be retained on the distal portion of the endoscope 150.

The lumen outlet 154 for protrusion of the retention loop 153 can be formed in the area of the attraction described with Figs. 7 and 10 in the peripheral part of the second distal portion 37 of the endoscope 150. The distal portion of the assist device 12 or endoscopic catheter can be kept stationary in an area under the imaging window 55, or on the peripheral surface in an area (dotted in Fig. 10) opposite to the area of the lighting windows 56 and 57. Note that the lumen outlet 154 is depicted in Fig. 10 in such an orientation as to direct the upper side of the steering of the steering section 21 in the downward direction for clarification.

Only one light source, or three or more may be incorporated in the endoscopes 11 and 150. Also, a light source may include an LED and a driver for driving the LED in place of the light guide optics 48 and 49 and the lighting lens.

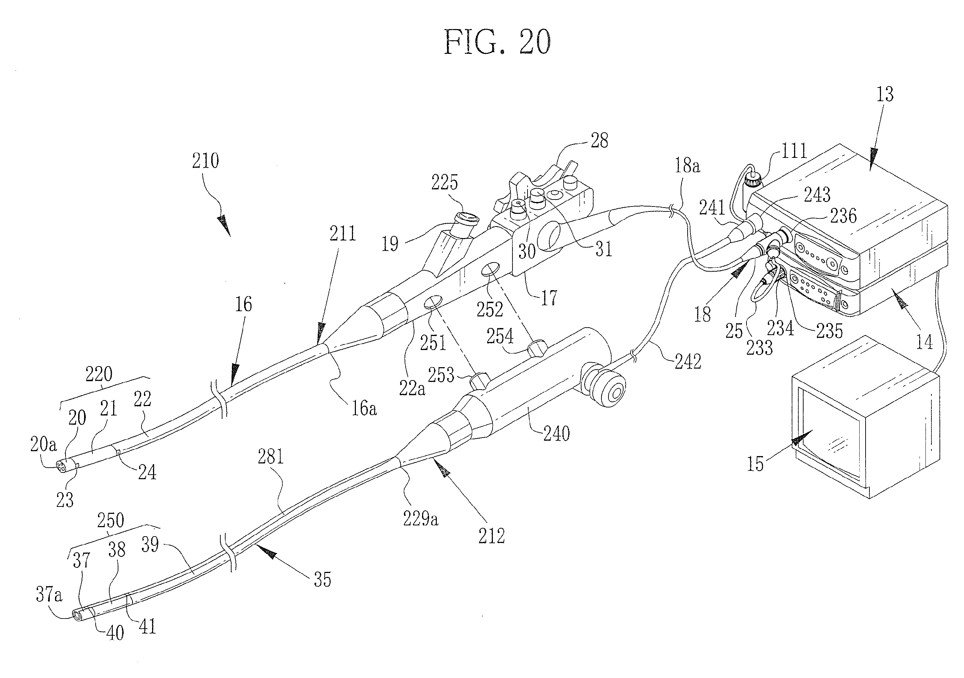

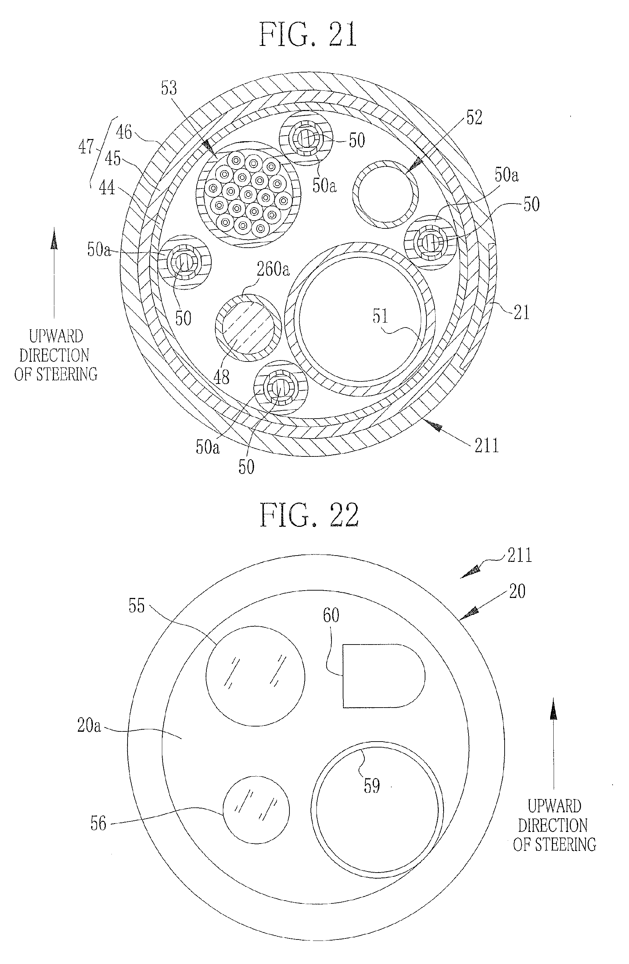

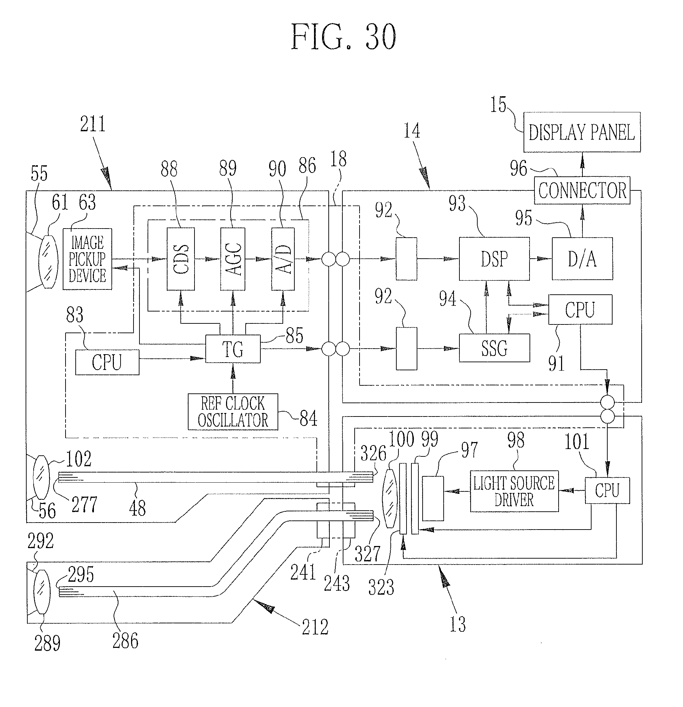

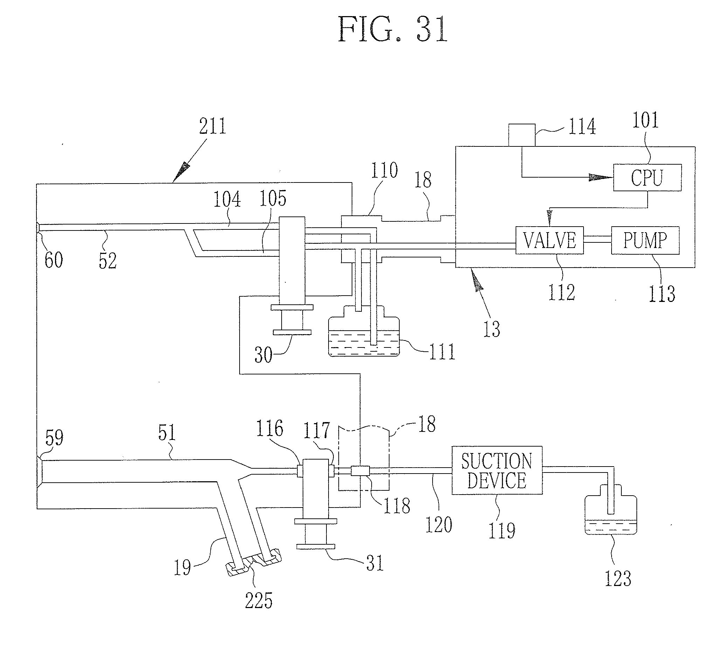

In Figs. 20 - 32, another preferred endoscope system 210 which is not part of the present invention is illustrated, which is for the purpose of removing a shadow of a forceps device in an endoscopic image in imaging of an object in a body. Elements of the above embodiments are designated with identical reference numerals. The endoscope system 210 includes a nasal endoscope 211, an assist device 212 or light delivery catheter, the light source apparatus 13, the processing apparatus 14 and the display panel 15. A proximal end 16a is included in the first elongated tube 16, and is connected with the grip portion 22a having a great diameter.

The handle section 17 includes the air/water supply button 30 and the suction button 31 in addition to the steering wheels 28. A sealing film 225 or channel lid is disposed in the proximal opening 19 in a removable manner. A sealing portion of the sealing film 225 has flexibility, includes slits or small pores, and is openable when pushed by a medical instrument. The sealing film 225 partially seals the proximal opening 19 and prevents fluid from flowing out of the proximal opening 19 through the instrument channel.

The first distal portion 20 includes a rigid tubular structure and relevant devices contained in the tubular structure. The devices include imaging optics, an image pickup device and lighting optics. The universal connector 18 includes the light guide coupler 25 and a video connector 234 or plug. There is a signal cable 233 of which the video connector 234 is secured to an end. An electric connector 235 or plug or socket is disposed in the front panel of the processing apparatus 14, and connected with the video connector 234. A light guide coupler 236 or socket is disposed in the front panel of the light source apparatus 13, and connected with the light guide coupler 25.

In the assist device 212, a grip portion 240 is attached to a proximal end 229a of the second elongated tube 35, and has a great diameter to facilitate manual grasping. A light guide cable 242 extends from the grip portion 240. An auxiliary light guide coupler 241 is disposed at an end of the light guide cable 242. An auxiliary light guide coupler 243 is an optical element associated with the light source apparatus 13. The auxiliary light guide coupler 241 is connected with the auxiliary light guide coupler 243 in a removable manner.

A second distal tube region 250 is included in the second elongated tube 35 of the assist device 212. Light guide optics are contained in a tube lumen which extends from the second distal tube region 250 to the proximal end 229a through the second elongated tube 35. The light guide optics transmit light from the light source apparatus 13 toward lighting optics disposed behind a lighting window on the second distal end surface 37a of the second elongated tube 35.

The grip portion 240 of the assist device 212 is engaged with the handle section of the endoscope 211 in a removable manner, and can be handled together. There are receiving holes 251 and 252 or receiving portions formed in the grip portion 22a of the endoscope 211. Engaging projections 253 and 254 or engaging portions are formed on the grip portion 240 of the assist device 212 for engagement with the receiving holes 251 and 252. A structure including the engaging projections 253 and 254 and the receiving holes 251 and 252 constitutes an engaging mechanism in a removable manner. The receiving holes 251 and 252 are recesses or cut-outs. A small flexible ridge is formed with each of the engaging projections 253 and 254 for engagement with the hole in a manner with a click. The receiving hole 252 is positioned from the receiving hole 251 at a predetermined distance in the axial direction of the first elongated tube 16. The engaging projection 254 is positioned from the engaging projection 253 at a predetermined distance in the axial direction of the second elongated tube 35. One of the receiving holes 251 and 252 and one of the engaging projections 253 and 254 operate for positioning. A remaining one of the receiving holes 251 and 252 and a remaining one of the engaging projections 253 and 254 operate for an anti-rotation purpose. Note that other structures can be used for the engagement, for example, a claw and a hole in combination, magnetic attraction with magnetic components, a strip for winding to retain, and the like.

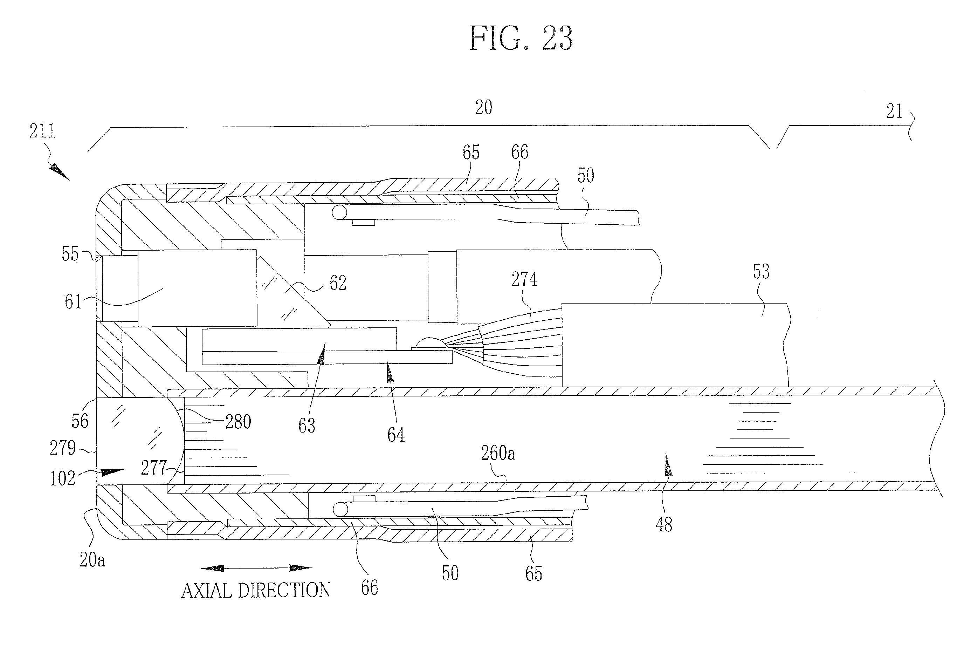

In Fig. 23, a part of the imaging optics 61 is disposed in the imaging window 55. Light emitted from the lighting window 56 travels to and is reflected by an object in the patient's body, and then becomes incident on the imaging optics 61. A prism 271 receives the object light from the imaging optics 61 and refracts the object light. A light receiving surface of an image pickup device 272 receives the object light from the prism 271. A circuit board 273 is connected with the image pickup device 272. Signal lines 274 of the multi-core cable 53 are connected with the circuit board 273.

The light guide optics 48 are contained in the steering section 21 of the endoscope 211. A plurality of optical fibres are bundled in a cylindrical shape. A protective tube 260a is formed from elastic material such as silicone resin, and wraps the optical fibres to constitute the light guide optics 48. The lighting lens 102 has an entrance surface 280. An exit end 277 of the light guide optics 48 is kept in contact with the entrance surface 280 inside the rubber layer 65. An exit surface 279 of the lighting lens 102 is located opposite to the entrance surface 280, and constitutes the lighting window 56. The lighting lens 102 is the lighting optics according to the invention. The exit end 277 of the light guide optics 48 is also kept stationary inside the rubber layer 65.

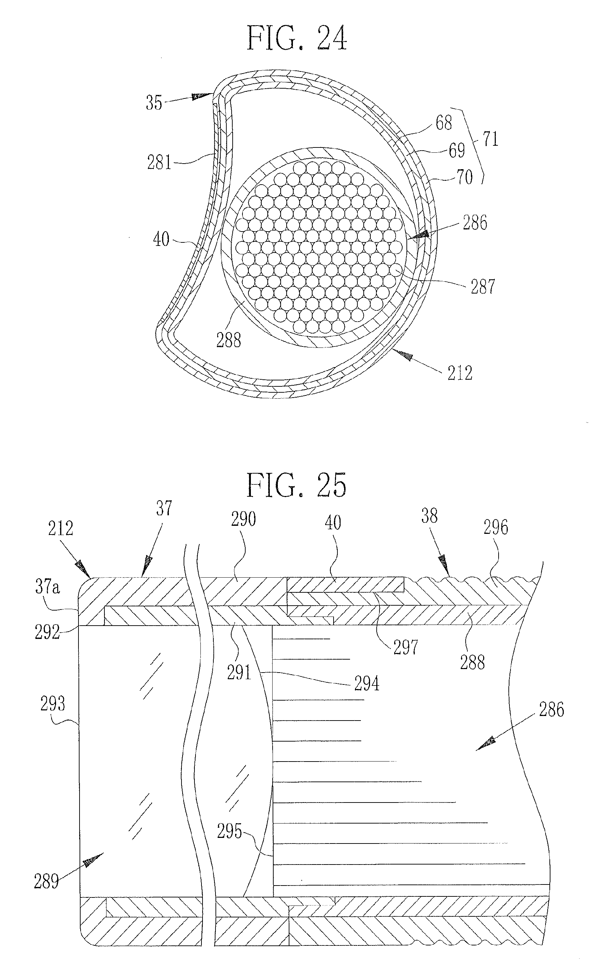

In Fig. 24, a longitudinal receiving surface 281 is formed in the second elongated tube 35 of the assist device 212, and has a shape with a concave surface for tight contact with a first distal tube region 220 of the endoscope 211. The receiving surface 281 causes the second elongated tube 35 to have a form of a crescent as viewed in a cross section. In the covering layer 70 of the receiving surface 281, the magnets 40 and 41 are fitted to have the receiving surface 281 for attraction of the magnets 23 and 24 of the endoscope 211. The shape of the second elongated tube 35 of the assist device 212 can be circular. Also, ferromagnetic components attractable magnetically may be used in place of the magnets 40 and 41.

Light guide optics 286 are contained in the assist device 212. A plurality of optical fibres 287 are bundled in a cylindrical shape. A protective tube 288 is formed from elastic material such as silicone resin, and wraps the optical fibres 287 to constitute the light guide optics 286. A diameter of the light guide optics 286 except for the protective tube 288 is set greater than a diameter of the light guide optics 48 of the endoscope 211.

In Fig. 25, an outer rigid ring 290 is a covering part of the second distal portion 37 of the assist device 212. A lens holder 291 is retained inside the second distal portion 37 in a stationary manner. A lighting lens 289 is retained in the lens holder 291 as lighting optics. An exit surface 293 of the lighting lens 289 is positioned in a lighting window 292 which is formed in the second distal end surface 37a. An entrance surface 294 of the lighting lens 289 is positioned opposite to the exit surface 293. An exit end 295 of the light guide optics 286 is disposed at the entrance surface 294. Note that the lighting lens 289 may include plural lens elements instead of a single lens element. In addition to the removal of a shadow with the lighting lens 289 in the assist device 212, it is possible to change a region of diffusion of a light lens on the entrance surface 294 between a narrow region for compensating for shortage in the light amount of the endoscope 211 and a wide region for enlarging the angle of the distribution.

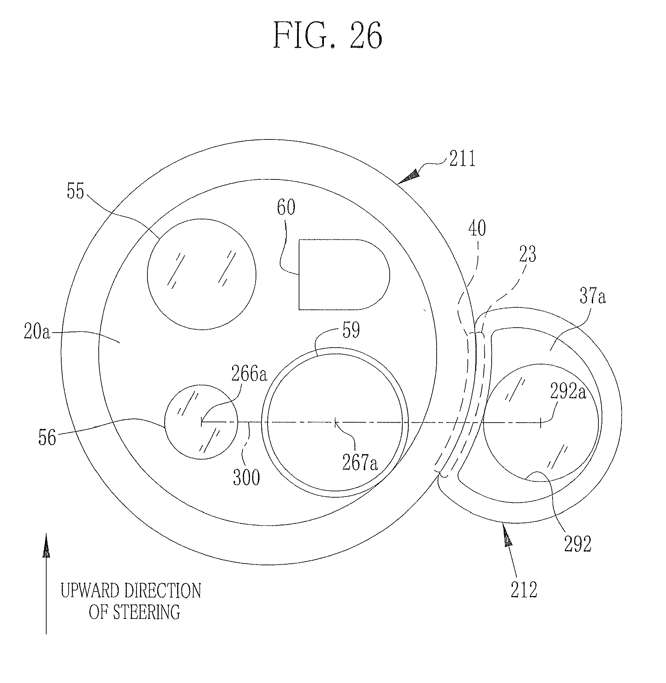

The steering section 38 has a covering layer 296. Two cut-outs 297 are formed in the covering layer 296, and receive the magnets 40 and 41. In Fig. 26, the magnets 40 and 41 are fitted in a peripheral part of the second distal tube region 250 as viewed with respect to the second distal end surface 37a in an arcuate form. The peripheral part is so determined as to attract the second distal tube region 250 of the assist device 212 to position the lighting window 292 in the second distal end surface 37a of the assist device 212 opposite to the lighting window 56 with reference to the distal opening 59 in the first distal end surface 20a of the endoscope 211.

Upon the magnetic attraction, the endoscope 211 and the assist device 212 are so positioned that the isolator 92 is positioned opposite to the lighting window 56 with reference to the distal opening 59 of the endoscope 21. Note that it is preferable to position a window centre point 292a of the lighting window 292 of the assist device 212 on a straight line 300 which passes a window centre point 266a of the lighting window 56 of the endoscope 211 and an opening centre point 267a of the distal opening 59.

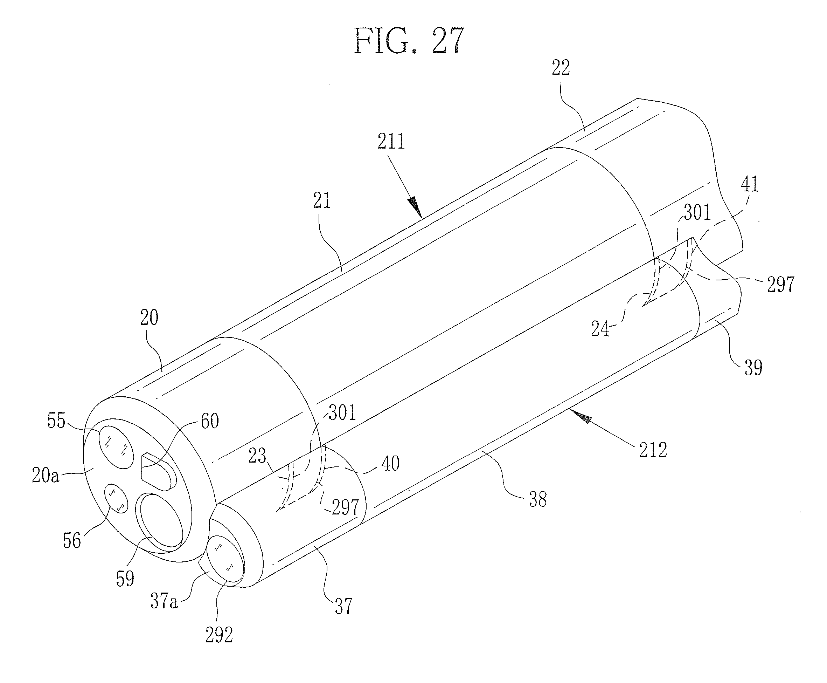

In Fig. 27, the magnets 40 and 41 of the assist device 212 are fitted in the cut-outs 297 formed in the covering layer 296, and are set to have a surface flush with the covering layer 296. Also, a cutout 301 is formed in the outer wall of the endoscope 211, and receives the magnets 23 and 24 to define their surface flush with the outer wall.

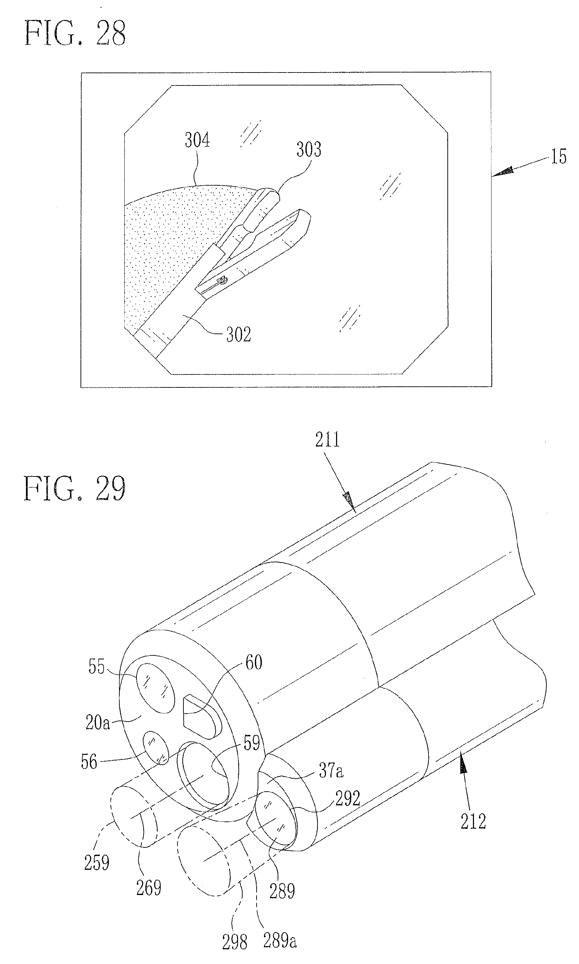

The distal opening 59 is disposed on the lower left side from the imaging window 55. In Fig. 28, an image of a forceps device 302 or treatment instrument appears on the display panel 15 in a form protruding from the lower left side. Also, the lighting window 56 is disposed on the right side from the distal opening 59 as viewed from the imaging window 55. A shadow due to the forceps device 302 and an instrument head 303 appears in the image. As the assist device 212 is combined for use, the lighting window 292 of the assist device 212 is positioned on the left side as viewed from the imaging window 55. A shadow 304 of the forceps device 302 and the instrument head 303 can be eliminated by lighting of the assist device 212 to the left side of the forceps device 302 and the instrument head 303.

Specifically with a large distribution of light from the assist device 212, a problem is likely to occur with shadows of a forceps device or its instrument head on a side opposite to the assist device 212 with reference to the distal opening 59. Let an axis 269 be a line where a forceps device moves out of the distal opening 59 of the endoscope 211. In Fig. 29, it is preferable for the lighting lens 289 to have such a distribution characteristic that a light flux 298 exited from the lighting lens 289 of the assist device 212 does not intersect with the axis 269 irrespective of an object distance in an imaging direction from the distal end surfaces 20a and 37a of the elongated tubes 16 and 35 of the endoscope 211 and the assist device 212. Note that a movable range 259 is defined as a range where the forceps device can be moved in and out. The lighting lens 289 has an optical axis 289a which is substantially parallel with the axis 269.

In the light source apparatus 13, a filter device 323 for limiting light is movable into and out of a light path of light from the illuminator 97, and limits a light amount of a component of a particular band of a wavelength in the white light from the illuminator 97.

Light from the illuminator 97 is adjusted by the aperture stop device 99 and the condensing lens 100. Entrance ends 326 and 327 are included in respectively the light guide optics 48 of the endoscope 211 and the light guide optics 286 of the assist device 212. The adjusted light becomes incident upon the entrance ends 326 and 327. Light emitted from the exit end 277 of the light guide optics 48 of the endoscope 211 is applied to an object in the body through the lighting lens 102 and the lighting window 56. Light emitted from the exit end 295 of the light guide optics 286 of the assist device 212 is applied to the object in the body through the lighting lens 289 and the lighting window 292.

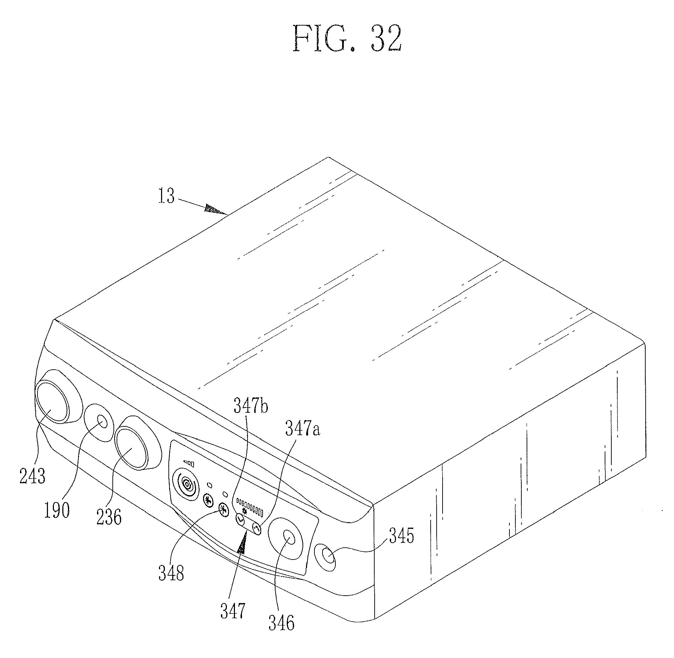

In Fig. 32, a power button 345 is disposed on the front of the light source apparatus 13 near to the right corner. The power button 345 is turned on and off to switch the supply of power to the light source apparatus 13 by use of a commercial power source. There are a lighting button 346, a light adjusting button group 347 and a light limiting button 348 arranged beside the power button 345. Also, the light guide coupler 236 is disposed on the front of the light source apparatus 13 for connection with the light guide coupler 25. The auxiliary light guide coupler 243 is disposed on the front of the light source apparatus 13 for connection with the auxiliary light guide coupler 241 of the assist device 212.

The lighting button 346 is a pushbutton for turning on and off the light source. The light adjusting button group 347 includes an increase button 347a and a decrease button 347b, and adjusts the light stepwise by control of a diameter of the aperture, for example in 10 steps. Note that the brightness can be adjusted by insertion of a light limiting filter, adjustment of current for a light source lamp, and the like. The light limiting button 348 is a pushbutton for turning on or off the limitation of light amount of a component of a predetermined band in the white light by use of a filter movable into or out of the light path.

Before imaging by use of the endoscope 211, the lamp of the light source is adjusted for brightness and a wavelength of light to be emitted according to information such as personal data of a patient, a result of the diagnosis, information of an object or tissue in his or her body. In the light source apparatus 13, the power button 345 is turned on, before the lighting button 346 is turned on. The light adjusting button group 347 is operated to adjust and optimize the brightness of the object. Then the light limiting button 348 is turned on or off as required.

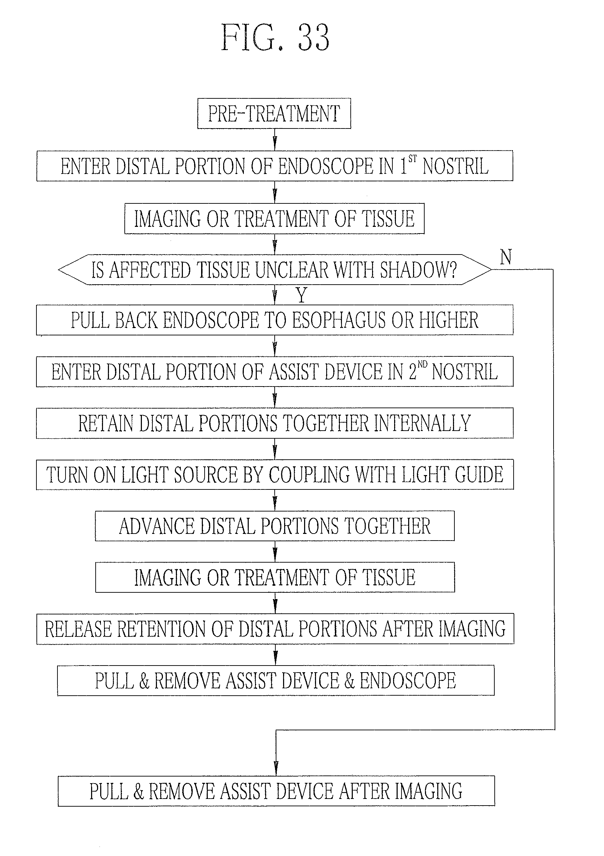

The operation of the embodiment which is not part of the present invention is hereinafter described by referring to Fig. 33. When a doctor discovers a lesion in a duodenum, stomach or other organs in the gastrointestinal tract, it is checked by use of the endoscope 211 whether treatment of the lesion is possible. If the treatment is possible, the lesion is treated by use of a forceps device or other medical instruments. Specifically, the forceps device is inserted in the proximal opening 19 of the endoscope 211 for an instrument head to protrude through the distal opening 59. A handle is positioned at a proximal end of the forceps device, and is slid outside the proximal opening 19 to move the instrument head back and force. In Fig. 28, shadows appear on the display panel 15 according to shapes of the forceps device and the instrument head. It is likely that a lesion cannot be clearly imaged due to the shadows. To solve this problem, the assist device 212 is used.

To combine the assist device 212 with the endoscope 211, at first a nasal tract in connection with the second nostril is anesthetized for the purpose of entering the second elongated tube 35 of the assist device 212 in the second nostril. The elongated tubes 16 and 35 are shifted relative to one another in order to position the distal end surfaces 20a and 37a equally in the elongated tubes 16 and 35, so that the magnet 23 of the endoscope 211 attracts the magnet 40 of the assist device 212 and the magnet 24 of the endoscope 211 attracts the magnet 41 of the assist device 212.

Then the grip portion 240 of the assist device 212 is secured to the grip portion 22a of the endoscope 211 next. To this end, the receiving holes 251 and 252 are engaged with the engaging projections 253 and 254. This facilitates the manual handling because the grip portion 240 of the assist device 212 can be grasped together with the grip portion 22a of the endoscope 211.

Then the auxiliary light guide coupler 241 of the assist device 212 becomes connected with the auxiliary light guide coupler 243 of the light source apparatus 13. The lighting button 346 remains turned on. There is a cap fitted on the auxiliary light guide coupler 243 for preventing leaking of light. The auxiliary light guide coupler 243 can be connected only after removing the cap. After the connection, the elongated tubes 16 and 35 are entered gradually.

As has been described with Fig. 26, the second distal end surface 37a of the assist device 212 is positioned on the left from the imaging window 55 because of the use of the assist device 212. The left side of the forceps device and instrument head can be illuminated to remove their shadows. If the instrument head remains open, a lesion in front of the instrument head can be illuminated to carry out the treatment in a good condition of observation with the endoscope.

After the treatment is finished, the medical instrument such as biopsy forceps device and snare instrument is removed from the proximal opening 19 of the endoscope 211. The second distal tube region 250 is disengaged from the first distal tube region 220 of the endoscope 211. Then the second elongated tube 35 and the first elongated tube 16 of the assist device 212 and the endoscope 211 are removed from the body, in the order of the second and then the first. Finally, the lighting button 346 is turned off. The auxiliary light guide coupler 241 of the assist device 212 is removed from the auxiliary light guide coupler 243 of the light source apparatus 13. Note that the assist device 212 can be used in addition from the initial step of the operation.

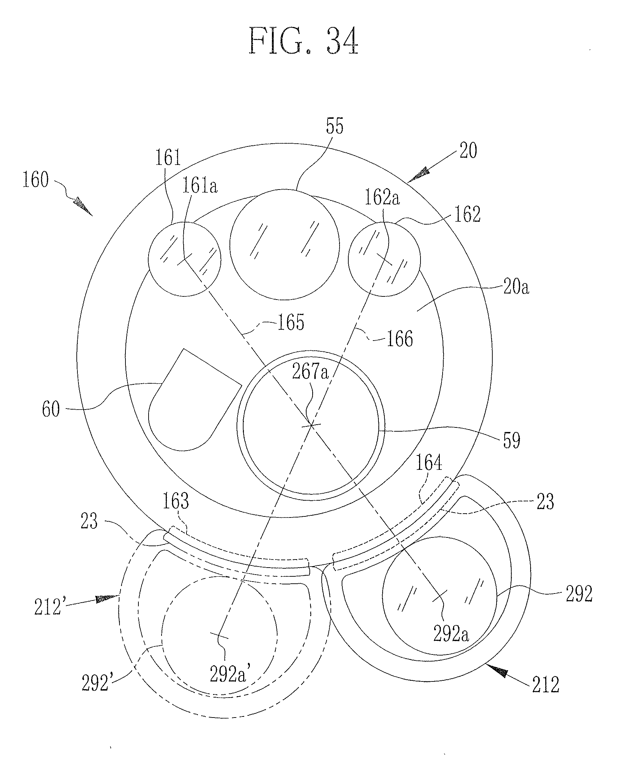

In the endoscope 211 of the above embodiment, only the lighting window 56 is formed in the first distal end surface 20a. In Fig. 34, one preferred nasal endoscope 160 which is not part of the present invention is illustrated, and includes two lighting windows 161 and 162 beside the imaging window 55. It is possible to dispose the lighting window 292 of the assist device 212 on a side opposite to the lighting window 161 with reference to the distal opening 59 of the endoscope 160, and to dispose a window centre point 292' of an assist device 212' or light delivery catheter on a side opposite to the lighting window 162 with reference to the distal opening 59. Thus, magnets or magnetic components 163 and 164 of the endoscope 211 are arranged in a range to retain the second distal tube region 250 of the assist device 212 at the two points. The magnets 163 and 164 are so disposed that the window centre point 292a of the lighting window 292 of the assist device 212 is positioned on a straight line 165 which passes a window centre point 161a of the lighting window 161 and the opening centre point 267a of the distal opening 59. Furthermore, it is possible to dispose the magnets 163 and 164 so that a window centre point 292a' of the lighting window 292' of the assist device 212' is positioned on a straight line 166 which passes a window centre point 162a of the lighting window 162 and the opening centre point 267a of the distal opening 59.

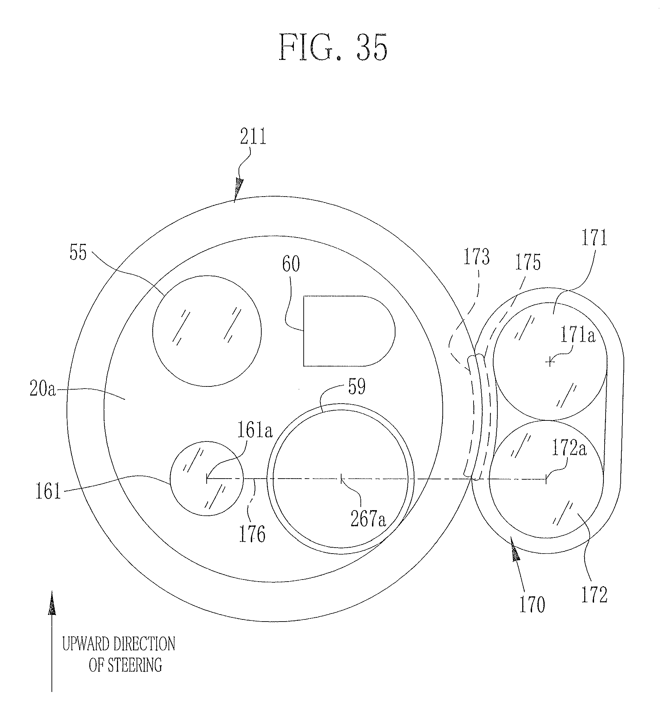

In the above embodiment, one lighting window is formed in the assist device 212. In Fig. 35, another preferred assist device 170 or light delivery catheter which is not part of the present invention includes two lighting windows 171 and 172. Two magnets or magnetic components 173 are disposed on the elongated tube of the endoscope 160, and arranged in the axial direction. Two magnets or magnetic components 175 are positioned on a periphery of the elongated tube of the assist device 170. The magnets 175 are attracted on the magnets 173 in such a position that a window centre point 172a of the lighting window 172 of the assist device 170 is positioned on a straight line 176 passing the window centre point 161 a of the lighting window 161 and the opening centre point 267a of the distal opening 59 and opposite to the lighting window 161 with reference to the distal opening 59.

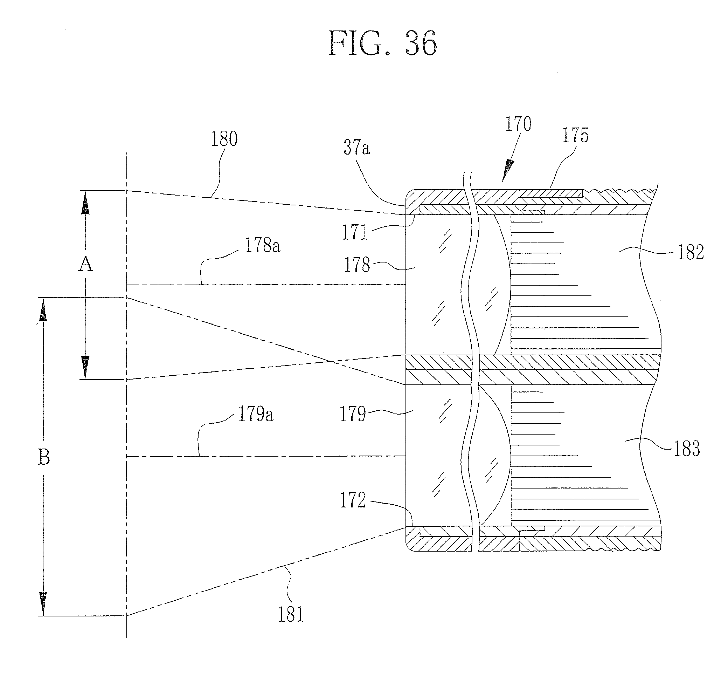

In Fig. 36, a lighting lens 178 is disposed in the lighting window 171 or spot lighting window of the assist device 170. There is created a spot area 180 illuminated by light from the lighting lens 178 in spot illumination or high beam illumination in a manner of suppressing diffusion. A lighting lens 179 is disposed in the lighting window 172 of which the window centre point 172a is positioned on the straight line 176. There is created a large area 181 illuminated by light from the lighting lens 179 in low beam illumination. Note that optical axes 178a and 179a of the lighting lenses 178 and 179 are parallel with one another.

In the embodiment of Fig. 36, a first area B illuminated through the lighting lens 179 at a predetermined irradiance is larger than a second area A illuminated through the lighting lens 178 at the equal predetermined irradiance, at any object distance from the second distal end surface 37a of the assist device 170 in the imaging direction. It is possible to remove a shadow of the forceps device by the low beam illumination and to obtain a high light amount of illumination because of the single lighting window 161 of the endoscope 211 in the high beam illumination. In Fig. 36, light guide optics 182 and 183 are illustrated.

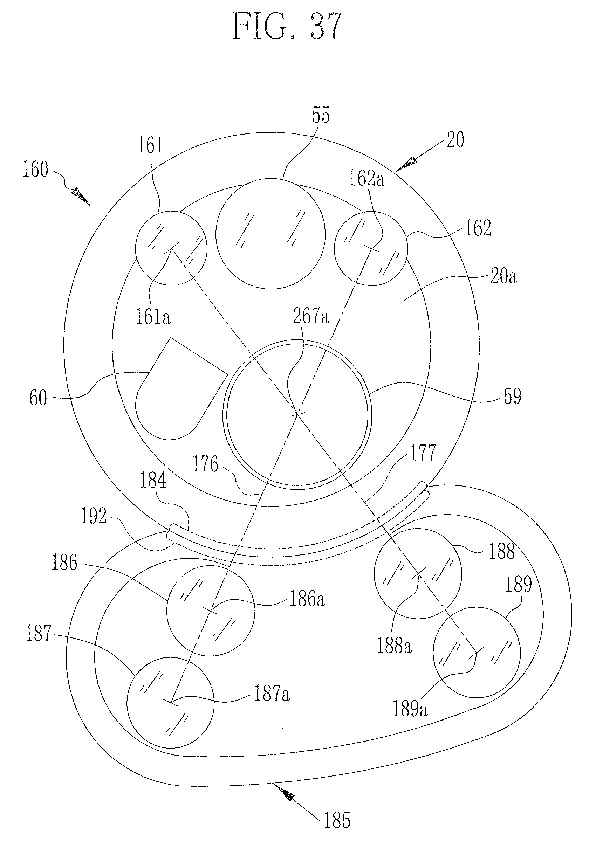

In Fig. 37, one preferred assist device 185 or light delivery catheter is illustrated, and has four lighting windows 186, 187, 188 and 189. A magnet or magnetic component 184 is disposed on the outside of the endoscope 160. A magnet or magnetic component 192 is disposed on the outside of the assist device 185, and attracts the magnet 184 at a single point. The lighting windows 186 and 187 of the assist device 185 become positioned opposite to the lighting window 162 of the endoscope 160 with reference to the distal opening 59. The lighting windows 188 and 189 of the assist device 185 become positioned opposite to the lighting window 161 of the endoscope 160 with reference to the distal opening 59.

Window centre points 186a and 187a of the lighting windows 186 and 187 of the assist device 185 are disposed on the straight line 176 which passes the window centre point 162a of the lighting window 162 of the endoscope 160 and the opening centre point 267a of the distal opening 59. Window centre points 188a and 189a of the lighting windows 188 and 189 of the assist device 185 are disposed on a straight line 177 which passes the window centre point 161 a of the lighting window 161 of the endoscope 160 and the opening centre point 267a of the distal opening 59. The lighting windows 186 and 188 nearer to the endoscope 211 operate for high beam illumination as spot lighting windows. The lighting windows 187 and 189 farther from the endoscope 211 operate for low beam illumination as diffusion lighting windows.

In the above embodiments, the assist devices 170, 185 and 212 are connected directly to the light source apparatus 13. Alternately, a coupler may be added to the light guide optics by branching off in the endoscope 211. The light guide optics of the assist devices 170, 185 and 212 can be connected with the coupler for transmitting light from the endoscope 211 to the assist devices 170, 185 and 212.

In the above embodiments, the light source for the endoscope 211 is used also for lighting in the assist devices 170, 185 and 212. However, two light source units may be contained in the light source apparatus 13 for the endoscope 211 and the assist devices 170, 185 and 212. For this structure, an auxiliary lighting button 190 is disposed in the light source apparatus 13 for turning on the lighting of the assist devices 170, 185 and 212. See Fig. 32.

Also, a second light source may be added for generating light for the assist devices 170, 185 and 212 discretely from the light source apparatus 13 for generating light for the endoscope 211.

In the above embodiments, the light source for the assist devices 170, 185 and 212 is associated with light guide optics. However, a light source may have a white LED. For this structure, a driver and an auxiliary connector are incorporated in each of the assist devices 170, 185 and 212. The driver drives the LED by connection of a signal line. The auxiliary connector is disposed at an end of a cable extending from a proximal end of the assist devices 170, 185 and 212, and is connected with an LED controller. The LED controller is connected with the processing apparatus 14, retrieves brightness information by communication with the CPU in the processing apparatus 14 upon creating a video signal, and controls the driver to optimize the brightness of the LED for the imaging condition. A lighting button is associated on the LED controller. The LED can be turned on and off by operating the lighting button. Note that the driver may be incorporated in the distal portion of the assist devices 170, 185 and 212 or in the LED controller. Also, it is possible to incorporate the LED controller in the light source apparatus 13 and connect the auxiliary connector with the light source apparatus 13.

Also, a white LED for lighting may be used in the endoscope 211 instead of the light guide optics. For this structure, the single LED controller can be used in connection with both of the endoscope 211 and the assist devices 170, 185 and 212. Alternatively, two LED controllers can be installed discretely for connection with the endoscope 211 and the assist devices 170, 185 and 212.

Furthermore, it is possible in an assist device or light delivery catheter to incorporate an instrument channel, a water jet channel and the like in addition to the lighting structure.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. An endoscope system having an endoscope (10, 150) including a first elongated tube (16), having a first distal portion (20), for entry in a first external nostril, an image pickup device (63) for picking up an image of an object through an imaging window (55) formed in a first distal end surface (20a) of said first distal portion, a first instrument channel (51), contained in said first elongated tube, for extending from said first distal portion toward a proximal portion (16a), a handle section (17) connected with said proximal portion, and a steering section (21) for steering said first distal portion at least in an upward or downward direction upon steering operation of a steering mechanism (28) disposed in said handle section, said endoscope system comprising:
an assist device (12, 140) including a second elongated tube (35), having a second distal portion (37), for entry in a second external nostril, and a second instrument channel (72), defined in said second elongated tube, for extending from a second distal end surface (37a) of said second distal portion in a proximal direction, said second instrument channel having a greater inner diameter than said first instrument channel;
a display panel (15) for operating according to an image signal of said image picked up by said image pickup device, and displaying said image in a state of orienting an upper side of steering of said steering section in said upward direction;
a retaining device (23, 24, 40, 41, 151) for retaining said second distal portion on said first distal portion removably to orient said first and second distal end surfaces in an equal direction, to cause said display panel to display an image of a medical instrument (302) through said second instrument channel in a partial area lower than a centre point of a display area thereof;
wherein said imaging window (55) is positioned eccentrically with reference to a centre point (P) of said first distal end surface (20a);
and further comprising two lighting windows (56, 57) formed in said first distal end surface (20a), for emitting light toward said object,
**characterized in that**
said retaining device (23, 24, 40, 41, 151) holds said assist device, such that said assist device is located at one of two areas defining an outer periphery of said first distal portion (20), in which said imaging window (55) and said two lighting windows are not disposed, said two areas being defined by a straight line which is tangential to circumferences of said two lighting windows and which is perpendicular to a central line passing a centre point of said imaging window (55) and said centre point (P) of said first distal end surface (20a).

2. An endoscope system as defined in claim 1, **characterized in that** said second elongated tube includes a rigid distal portion (37), a steering section (38), and a flexible tube (39).

## Patentansprüche

1. Endoskopsystem, das ein Endoskop (10, 150) aufweist, das eine erste längliche Röhre (16), aufweisend einen ersten Distalabschnitt (20), zum Einführen in ein erstes äußeres Nasenloch, eine Bildaufnahmeeinrichtung (63) zum Aufnehmen eines Bildes eines Objekts durch ein Bilderfassungsfenster (55), das in einer ersten distalen Endoberfläche (20a) des ersten Distalabschnitts gebildet ist, einen ersten Instrumentenkanal (51), der in der ersten länglichen Röhre aufgenommen ist, zum Erstrecken von dem ersten Distalabschnitt zu einem Proximalabschnitt (16a), einen Griffbereich (17), der mit dem Proximalabschnitt verbunden ist, und einen Lenkbereich (21) zum Lenken des ersten Distalabschnitts zumindest in einer aufwärtigen oder abwärtigen Richtung bei der Lenkoperation eines Lenkmechanismus (28), der in dem Griffbereich angeordnet ist, enthält, das Endoskopsystem umfassend:
eine Unterstützungseinrichtung (12, 140), die eine zweite längliche Röhre (35), aufweisend einen zweiten Distalabschnitt (37), zum Einführen in ein zweites äußeres Nasenloch, und einen zweiten Instrumentenkanal (72), der in der zweiten länglichen Röhre definiert ist, zum Erstrecken von einer zweiten distalen Endoberfläche (37a) des zweiten Distalabschnitts in eine proximale Richtung, enthält, wobei der zweite Instrumentenkanal einen größeren Innendurchmesser als der erste Instrumentenkanal aufweist;
einen Anzeigeschirm (15) zum Operieren entsprechend einem Bildsignal des Bildes, das durch die Bildaufnahmeeinrichtung aufgenommen wird, und zum Anzeigen des Bildes in einem Zustand eines Ausrichtens einer Oberseite einer Lenkung des Lenkabschnitts in die aufwärtige Richtung;
eine Festhalteeinrichtung (23, 24, 40, 41, 151) zum lösbaren Festhalten des zweiten Distalabschnitts an dem ersten Distalabschnitt, um die ersten und zweiten distalen Endoberflächen in eine gleiche Richtung auszurichten, um zu bewirken, dass der Anzeigeschirm ein Bild eines medizinischen Instruments (302) durch den zweiten Instrumentenkanal in einer teilweisen Zone, die niedriger als ein Mittelpunkt seiner Anzeigezone liegt, anzeigt;
wobei das Bilderfassungsfenster (55) in Bezug auf einen Mittelpunkt (P) der ersten distalen Endoberfläche (20a) exzentrisch positioniert ist;
und ferner umfassend zwei Beleuchtungsfenster (56, 57), die in der ersten distalen Endoberfläche (20a) gebildet sind, um Licht zu dem Objekt hin zu emittieren,
**dadurch gekennzeichnet, dass**
die Festhalteeinrichtung (23, 24, 40, 41, 151) die Unterstützungseinrichtung so hält, dass die Unterstützungseinrichtung an einer von zwei Zonen, die eine äußere Peripherie des ersten Distalabschnitts (20) definieren, in der das Bilderfassungsfenster (55) und die zwei Beleuchtungsfenster nicht angeordnet sind, lokalisiert ist, wobei die zwei Zonen durch eine gerade Linie definiert sind, die tangential zu Umfängen der zwei Beleuchtungsfenster ist, und die senkrecht zu einer Mittellinie ist, die durch einen Mittelpunkt des Bilderfassungsfensters (55) und den Mittelpunkt (P) der ersten distalen Endoberfläche (20a) läuft.

2. Endoskopsystem gemäß Anspruch 1, **dadurch gekennzeichnet, dass** die zweite längliche Röhre einen starren Distalabschnitt (37), einen Lenkbereich (38) und eine flexible Röhre (39) enthält.

## Revendications

1. Système endoscopique comportant un endoscope (10, 150) comprenant un premier tube allongé (16), ayant une première portion distale (20) pour une entrée dans une première narine externe, un dispositif capteur d'image (63) pour capter une image d'un objet à travers une fenêtre d'imagerie (55) formée dans une première surface d'extrémité distale (20a) de ladite première portion distale, un premier canal instrumental (51) contenu dans ledit premier tube allongé pour s'étendre de ladite première portion distale vers une portion proximale (16a), une section de poignée (17) raccordée à ladite portion proximale, et une section de guidage (21) pour guider ladite première portion distale au moins dans une direction ascendante ou descendante lors de l'opération de guidage d'un mécanisme de guidage (28) disposé dans ladite section de poignée, ledit système endoscopique comprenant :
un dispositif d'assistance (12, 140) comprenant un second tube allongé (35) ayant une seconde portion distale (37) pour une entrée dans une seconde narine externe et un second canal instrumental (72) défini dans ledit second tube allongé, pour s'étendre d'une seconde surface d'extrémité distale (37a) de ladite second portion distale dans une direction proximale, ledit second canal instrumental ayant un diamètre interne plus grand que celui du premier canal instrumental ;
un panneau d'affichage (15) opérant selon un signal d'image de ladite image captée par ledit dispositif capteur d'image et affichant ladite image dans un état d'orientation d'une face supérieure de guidage de ladite section de guidage dans ladite direction ascendante ;
un dispositif de retenue (23, 24, 40, 41, 151) pour retenir ladite seconde portion distale sur ladite première portion distale afin d'orienter de manière amovible lesdites première et seconde surfaces d'extrémité distales dans une même direction afin d'amener ledit panneau d'affichage à afficher une image d'un instrument médical (302) à travers ledit second canal instrumental dans une zone partielle plus basse qu'un point central de sa zone d'affichage ;
dans lequel ladite fenêtre d'imagerie (55) est positionnée de manière excentrique par rapport à un point central (P) de ladite première surface d'extrémité distale (20a) ;
et comprenant en outre deux fenêtres d'éclairage (56, 57) formées dans ladite première surface d'extrémité distale (20a) pour émettre de la lumière vers ledit objet,
**caractérisé en ce que** :
ledit dispositif de retenue (23, 24, 40, 41, 151) soutient ledit dispositif d'assistance de sorte que ledit dispositif d'assistance soit situé dans l'une de deux zones définissant une périphérie externe de ladite première portion distale (20), dans laquelle ladite fenêtre d'imagerie (55) et lesdites deux fenêtres d'éclairage ne sont pas disposées, lesdites deux zones étant définies par une ligne droite qui est tangentielle aux circonférences desdites deux fenêtres d'éclairage et qui est perpendiculaire à une ligne centrale passant par un point central de ladite fenêtre d'imagerie (55) et par ledit point central (P) de ladite première surface d'extrémité distale (20a).

2. Système endoscopique selon la revendication 1, **caractérisé en ce que** ledit second tube allongé comprend une portion distale rigide (37), une section de guidage (38) et un tube souple (39).
